# EUROPEAN PATENT APPLICATION

(11) **EP 2 692 866 A1**
(43) Date of publication of application: **05.02.2014**
(21) Application number: 12765339.2
(22) Date of filing: 31.03.2012
(51) Int. Cl.: C12N 15/29, C07K 14/415, C12N 15/82, C12N 5/04, C12N 1/21, A01H 5/00, C12R 1/01

(54) **GENETIC ENGINEERING METHOD AND MATERIAL FOR INCREASING PLANT YIELD**

(30) Priority: 31.03.2011 CN 201110081766
(71) Applicant: Shanghai Institutes for Biological Sciences, Chinese Academy of Sciences, Shanghai 200031 (CN)
(72) Inventor: CHEN, Xiaoya, Shanghai 200031 (CN); XU, Bing, Shanghai 200031 (CN); LIN, Zhiping, Shanghai 200031 (CN); WANG, Lingjian, Shanghai 200031 (CN); SHANGGUAN, Xiaoxia, Shanghai 200031 (CN); SHAN, Chunmin, Shanghai 200031 (CN)
(74) Representative: Osha Liang SARL
(86) International application number: PCT/CN2012/073393
(87) International publication number: WO 2012/130174

(57) **Abstract**

The present invention relates to genetic engineering methods and materials for improving plant yields. The present invention discloses that upregulating EXPA1 gene expression or activity of EXPA1 polypeptide, or co-expression of GhRDL1 and GhEXPA1 gene can improve plant traits. These methods have applications in crops and flower productions.

## Description

### TECHNICAL FIELD

This invention relates to plant bioengineering and plant improvement genetic engineering. Particularly, this invention relates to genetic engineering methods and materials for improving plant yield.

### BACKGROUND

Cotton is an important economy crop. Cotton fibers are important materials for the textile industry. In the past 3 years, world production of cotton has decreased. In 2009, the total cotton production in the world was 21.9 million tons, to which China contributed 6.4 million tons. This is the lowest production level in the past 6 years. The current situations make it favorable for cotton production. Because the inventory of cotton is low, the prices of cotton have climbed to the highest level in 50 years. At the same time, the cotton textile industry is demanding higher quality cottons, such as longer fibers, tougher, and more slender and even fibers. Improving the cotton quality and production is very important. This is a major objective for cotton cultivation research.

Cotton fibers are single-cell fibers that result from differentiation and growth of embryonic epithelial cells. The development process can be divided into four stages: fiber development starting period, elongation period, secondary cell wall thickening period, and maturation period. Among these, the elongation period and secondary cell wall thickening period overlap. Among these four stages, fiber cells undergo shape and structure changes, accompanied by important physiological and biochemical processes. During that time, a large number of genes participate in the regulation of fiber development.

Using cDNA array and RT-PCR, inventors of the present invention had previously identified RDL1 gene that is specifically expressed cotton fiber. This RDL1 gene is homologous to the RD22 gene of Arabidopsis thaliana. Cotton GhRDL1 gene is highly expressed in cotton fibers at 3-15 days post anthesis (DPA), and this expression decreases rapidly around 18 DPA. Therefore, RDL1 probably plays a role in the elongation of cotton fibers. Prior patent application (CN 200810033537.2; PCT/CN2009/070355) disclose: overexpression of RDL1 gene in cotton results in a phenotype with larger seeds and longer fibers. Results from analysis of transgenic arabidopsis also indicate: RDL1 gene overexpression leads to increased volumes in seeds. The superior larger seeds would be valuable in the development and use of crops, such as food crops, oil crops and fruit crops.

Crop seeds are important materials in the crop, cotton, and oil agriculture and industry. There is an urgent need for methods that can improve crop seed characteristics in order to have improved seed qualities and yields for food, cotton, and oil crops. In another aspect, such methods can also increase the number of flowers to increase the value of ornamental flowers.

### SUMMARY OF INVENTION

An objective of the invention is to provide a novel genetic engineering methods and material for improving plant yield.

In the first aspect of the invention, a method for improving plant traits is provided. The method comprises increasing the expression of *EXPA1* gene or the activity of EXPA1 polypeptide in the said plant.

In another preferred embodiment, wherein the increasing is achieved by introducing and expressing *EXPA1* gene in the said plant.

In another preferred embodiment, the method further comprises: increasing the expression of *RDL1* gene or the activity of RDL1 polypeptide in the said plant.

In another preferred embodiment, wherein the increasing is achieved by introducing and expressing *RDL1* gene in the said plant.

In another preferred embodiment, the said EXPA1 polypeptide is selected from one or more of the followings:
(a) a polypeptide of which sequence is shown as SEQ ID NO: 8 or 10;
(b) a polypeptide derived from that in (a), having one or more (e.g., 1-100, preferably 1-80, more preferably 1-50, more preferably 1-30, more preferably 1-20, or more preferably 1-10, for example, 1, 2, 3, 4, 5, 6, 7, 8, 9, or 10) amino acid substitutions, deletions, or additions in the sequence defined in (a) and possessing the plant traits improving function.

In another preferred embodiment, the said EXPA1 polypeptide is selected from one or more of the followings:
(a) a polypeptide comprising the consecutive amino acid sequence found at positions 197-258 of the EXPA1 protein;
(b) a polypeptide derived from that in (a), having one or more (e.g., 1-100, preferably 1-80, more preferably 1-50, more preferably 1-30, more preferably 1-20, or more preferably 1-10, for example, 1, 2, 3, 4, 5, 6, 7, 8, 9, or 10) amino acid substitutions, deletions, or additions in the sequence defined in (a) and possessing the plant traits improving function.

In another preferred embodiment, the said RDL1 polypeptide is selected from one or more of the followings:
(a1) a polypeptide of which sequence is shown as SEQ ID NO: 2, 4, or 6;
(b1) a polypeptide derived from that in (a1), having one or more (e.g., 1-100, preferably 1-80, more preferably 1-50, more preferably 1-30, more preferably 1-20, or more preferably 1-10, for example, 1, 2, 3, 4, 5, 6, 7, 8, 9, or 10) amino acid substitutions, deletions, or additions in the sequence defined in (a1) and possessing the plant traits improving function.

In another preferred embodiment, the said *EXPA1* gene comprises the polynucleotide sequence as one or more of the followings:
(a2) the polynucleotide sequence shown in SEQ ID NO: 7 or 9 (or the sequence described in GenBank ID AF043284 or DQ204495);
(b2) the sequence of a polynucleotide that can hybridize with the sequence defined in (a2) under stringent conditions.
(c2) a sequence having in increasing order of preference 40%, 50%, 60%, 70%, 75%, 80%, 85%, 90%, 92%, 95%, 96%, 98% or more sequence identity to the sequence defined in (a2).

In another preferred embodiment, the said *RDL1* gene comprises the polynucleotide sequence as one or more of the followings:
(a3) the polynucleotide sequence shown in SEQ ID NO: 1, 3, or 5 (or the sequence described in GenBank ID AY072821, AY641990, AY641991);
(b3) the sequence of a polynucleotide that can hybridize with the sequence defined in (a3) under stringent conditions.
(c3) a sequence having in increasing order of preference 40%, 50%, 60%, 70%, 75%, 80%, 85%, 90%, 92%, 95%, 96%, 98% or more sequence identity to the sequence defined in (a3).

In another preferred embodiment, the method comprises:
(1) providing a construct, wherein the construct comprises an EXPA1 polynucleotide expression cassette; and
(2) introducing the construct into a plant, thereby improving the plant traits.

In another preferred embodiment, the said construct in step (1) of the above method further comprises: an RDL1 polypeptide expression cassette, wherein the RDL1 polypeptide expression cassette and the EXPA1 polypeptide expression cassette are in the same construct or in different constructs.

In another preferred embodiment, the said construct is an expression vector.

In another preferred embodiment, in step (1), the said EXPA1 polypeptide expression cassette comprises (5'→3'): a promoter sequence, *EXPA1* gene sequence, and a terminator.

The said RDL1 polypeptide expression cassette comprises (5'→3'): a promoter sequence, *RDL1* gene sequence, and a terminator; or

In another preferred embodiment, all elements in the expression cassette are operably linked.

In another preferred embodiment, the promoter is CaMV 35S promoter.

In another preferred embodiment, the terminator is Nos PolyA terminator.

In another preferred embodiment, step (2) comprises:
(a) transfecting the said construct into agrobacterium to obtain construct-carrying agrobacterium; and
(b) contacting plant cell, tissue, or organ with the agrobacterium obtained in step (a) to introduce the construct into plant.

In another preferred embodiment, the said method further comprises:
(c) selecting plant cell, tissue, or organ that has been transfected with the construct; and
(d) regenerate the plant from the plant cell, tissue, or organ selected in step (c).

In another preferred embodiment, after upregulation of the *EXPA1* gene expression, the plant trait improvement comprises: increasing seed fiber length, or increasing branching numbers ; or increasing seed volumes, increasing seed weights, increasing fruiting numbers, or increasing yields.

In another preferred embodiment, after upregulation of the *EXPA1* gene and *RDL1* gene expressions, the plant traits improvement comprises: seed volumes increase, seed weights increase, seed fiber length increase, or seed fiber strength increase; or branching numbers increase, flower numbers increase, fruiting numbers increase, growth rate increase, budding acceleration, biomass increase, or yields increase.

In another preferred embodiment, the traits are agronomic traits; more preferably, the traits are yield-related trait.

In another preferred embodiment, wherein the plant is cotton and the said agronomic traits includes: cotton lints increase; cotton fiber length increase; cotton fiber strength increase; cotton yield increase or cotton fiber quality improve.

In another aspect of the invention, the invention provides the transgenic plants obtained by the above described method or hybrid offsprings of the said transgenic plant. When compared to control plants, these transgenic plants or offsprings have improved traits.

In another aspect of the invention, the invention provides constructs that comprise EXPA1 protein expression cassette.

In another preferred embodiment of the invention, the constructs further comprise RDL1 protein expression cassette.

In another aspect of the invention, the invention provides the use of the said constructs in improving plant traits.

In another preferred embodiment of the invention, the said constructs are used to transfect plants, resulting in plants with *EXPA1* gene over-expression or both *RDL1* and *EXPA1* gene over-expression, thereby improving the plant traits.

In another aspect of the invention, the invention provides host cells that comprise the said constructs.

In another aspect of the invention, the invention provides uses of the transgenic plants obtained by above-described methods, wherein the uses are for producing plant seeds having improved traits.

In another aspect of the invention, the invention provides a gene combination for improving plant traits, wherein the combination comprises *RDL1* gene and *EXPA1* gene.

In another aspect of the invention, the invention provides the uses of the said gene combination for improving plant traits.

Based on the disclosure in this application, other aspects of the invention will be apparent to one skilled in the art.

### BRIEF DESCRIPTION OF THE DRAWINGS

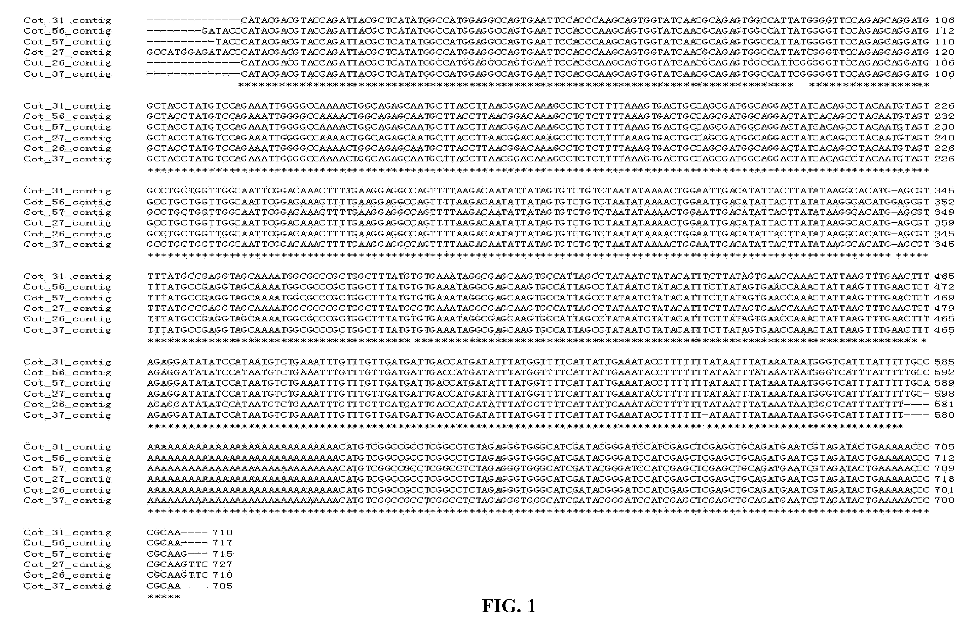

FIG. 1 shows the cotton *GhEXPA1* gene screened out by yeast two-hybrid screening.

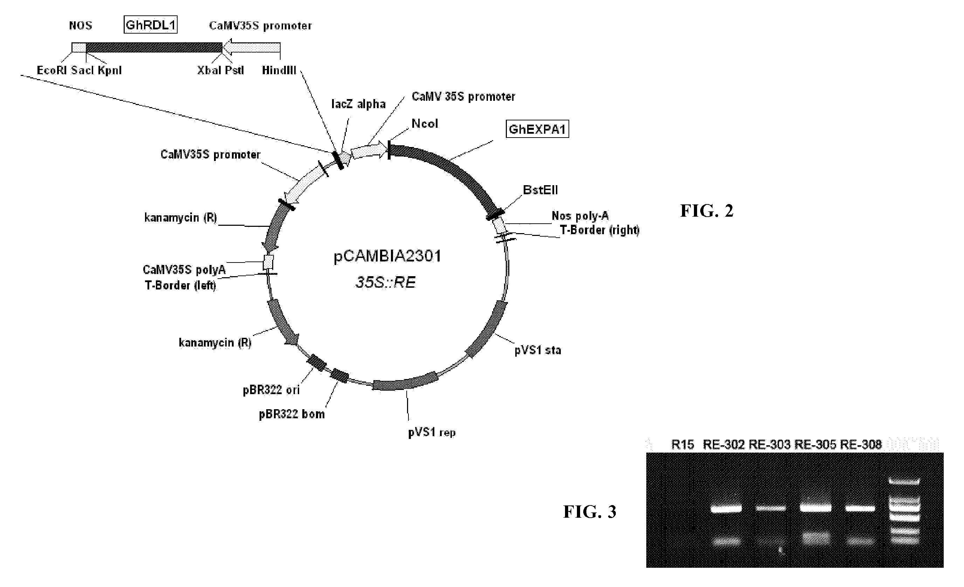

FIG. 2 shows the construction of the transfection vector of *35S::GhRDL1 35S::GhEXPA1.*
FIG. 3 shows molecular characterization of transgenic cottons containing *35S::GhRDL1 35S::GhEXPA1.* Since the vector does not include *GUS* gene, *GhRDL1* and *GhEXPA1* are endogenous genes in cotton, the gene detected by genomic PCR is the *Kanamycin* gene (*NPTII*) (the amplified fragment has 680 bp). All four cotton plants show positive results for the transgene.

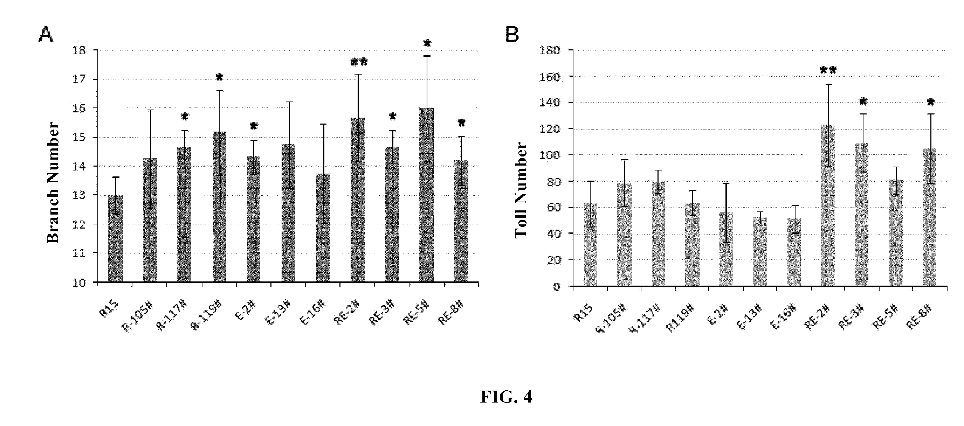

FIG. 4 shows branching numbers (left panel) and boll numbers (right panel) of *35S::GhRDL1 35S::GhEXPA1*transgenic cotton. The cotton strains analyzed here are: wild-type R15 and *GhRDL1* transgenic cotton (R-105#, R-117# and R-119#), *GhEXPA1* transgenic cotton (E-202#, E-213#, E-216#, and E-218#), and *GhRDL1 GhEXPA1* co-expression transgenic cotton (RE-302#, RE-303#, RE-305#, and RE-308#). Statistic analysis is based on t-test compared with R15, *: *p*<0.05; **: *p*<0.01.

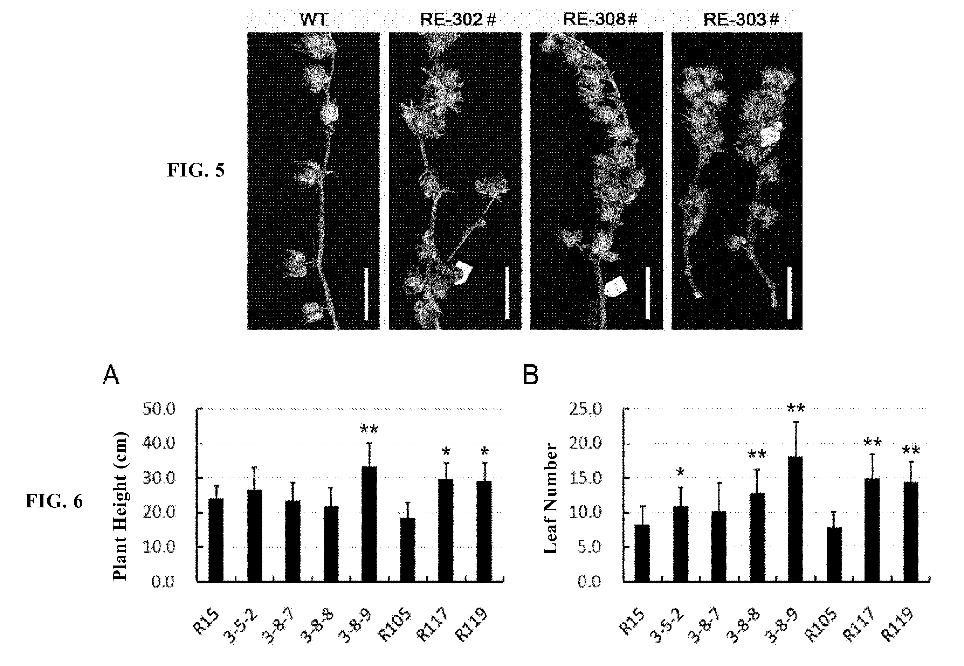

FIG. 5 shows the fruitings of *35S::GhRDL1 35S::GhEXPA1* transgenic cotton. WT: R15.
FIG. 6 shows plant heights and leave numbers of *35S::GhRDL1 35S::GhEXPA1* transgenic cotton. In the figure, WT indicates R15 wild-type cotton; R117 and R119 indicate *GhRDL1* transgenic cotton individual plant. "3-X-X (wherein X is an integer from 1 to 9)" indicates *35S::GhRDL1 35S::GhEXPA1* transgenic cotton individual plant. Date of analysis is 2011-02-21.

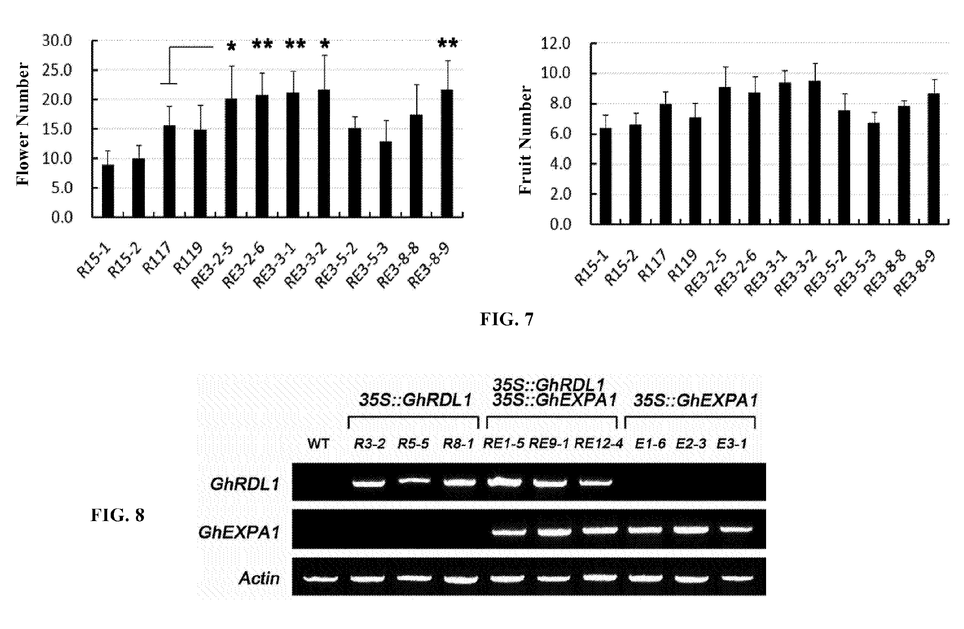

FIG. 7 shows flower bud numbers and fruit branch numbers of several *35S::GhRDL1 35S::GhEXPA1* transgenic cotton plants. In the figure, WT indicates R15 wild-type cotton; R117 and R119 indicate *GhRDL1* transgenic cotton individual plant; RE3-X-X indicates *35S::GhRDL1 35S::GhEXPA1* transgenic cotton individual plant. Date of analysis is 2011-03-28.
FIG. 8 shows molecular characterization of *35S::GhRDL1 35S::GhEXPA1* transgenic Arabidopsis thaliana. In the figure, R3-2, R5-5, and R8-1 are *GhRDL1* transgenic Arabidopsis thaliana; E1-6, E2-3, and E3-1 are *GhEXPA1* transgenic Arabidopsis thaliana; and RE1-5, RE9-1, and RE12-4 are *35S::GhRDL1 35S::GhEXPA1* transgenic Arabidopsis thaliana.

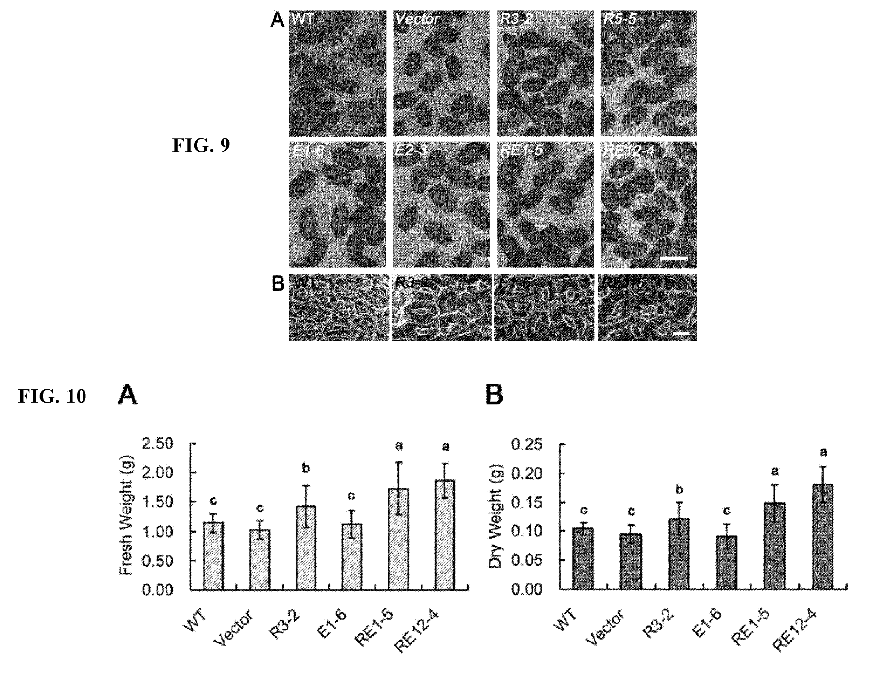

FIG. 9 shows seed size analysis of *35S::GhRDL1 35S::GhEXPA1* transgenic Arabidopsis thaliana.
   A shows mature seeds of Arabidopsis thaliana. WT, wild type; Vector, empty vector (*pCAMBIA2301*); transgenic plants: R3-2 and R5-5, *GhRDL1* transgenic Arabidopsis thaliana; E1-6 and E2-3, *GhEXPA1* transgenic plant; and RE1-5 and RE12-4, *GhRDL1* and *GhEXPA1* co-expression transgenic plant. Bar = 500 µm.
   B, scanning electromicroscope image of mature seed epithelial cells of Arabidopsis thaliana. Bar = 20 µm.
FIG. 10 shows biomass analysis of *35S::GhRDL1 35S::GhEXPA1* transgenic Arabidopsis thaliana. A, fresh weight; B, dry weight.
FIG. 11 shows the analysis of the growth of transgenic Arabidopsis thaliana.
   A, phenotype analysis of plant growth stage: 21-, 26- and 33-DAG (DAG stands for days after germination); 21-DAG plant shows lotus-seat leaves; 26-DAG plant shows rosette leaves with extended stems; and 33-DAG plant shows stem and silique. The plant strains (lines) are: wild-type, vector control, *R3-2, E1-6, RE1-5,* and *RE12-4.* The scale bar is 2 cm.
   B, time dependent curve of height changes of the stem during the plant growth 18 to 46-DAG. The plant strains (lines) are: wild-type, *R3-2, E1*-6, and *RE12-4.* Data shown are from 10 plants for each lines.

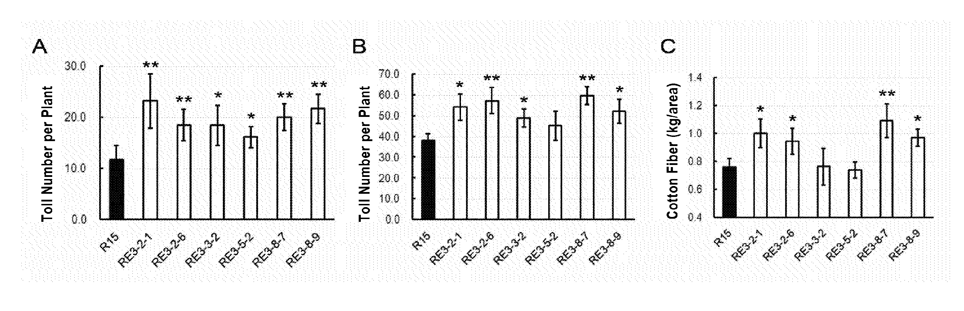

FIG. 12 shows the analysis of the cotton boll numbers and cotton lint yields of *35S::GhRDL1 35S::GhEXPA1* transgenic cotton.
   A, cotton boll numbers of *35S::GhRDL1 35S::GhEXPA1* transgenic cotton, wherein WT indicates wild-type R15 cotton; RE3-X-X indicates *35S::GhRDL1 35S::GhEXPA1* transgenic cotton individual plant. These data are average values of 20 plants. Cotton planting location: Sanya, Hainan, China.
   B, cotton boll numbers of *35S::GhRDL1 35S::GhEXPA1* transgenic cotton, wherein WT indicates wild-type R15 cotton; RE3-X-X indicates *35S::GhRDL1 35S::GhEXPA1* transgenic cotton individual plant. These data are average values of 30 plants. Cotton planting location: Songjiang, Shanghai, China.
   C. cotton lint yields of *35S::GhRDL1 35S::GhEXPA1* transgenic cotton (unit is based on planting area), wherein WT indicates wild-type R15 cotton; RE3-X-X indicates *35S::GhRDL1 35S::GhEXPA1* transgenic cotton individual plant. These data are average values of 3 areas. Cotton planting location: Songjiang, Shanghai, China. Note: Experiments in Sanya, Hainan and Songjiang, Shanghai are duplicated.

### DETAILED DESCRIPTION

The inventors of the present invention, after extensive research, found that expressing the *GhEXPA1* gene or simultaneously expressing the *GhRDL1* gene and the *GhEXPA1* gene can significantly improve plant traits. Therefore, the invention is valuable in applications to increase plant flower numbers and fruit numbers, and to improve plant traits.

### Definitions

### Polypeptide(s)/Protein(s)

The terms "polypeptide" and "protein" are used interchangeably herein and refer to amino acids in a polymeric form of any length, linked together by peptide bonds.

### Polynucleotide(s)/Nucleic acid(s)/Nucleic acid sequence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

As used herein, the term "plant" includes any kind of plants, as long as the plants are suitable for gene transformation applications (transgenic manipulations), such as a variety of crops, flower plants, or forestry plants. The plants can be, for example: dicotyledons, monocotyledons, or gymnosperms. Examples include, but not limited to: cruciferous Brassica cabbage, Chinese cabbage, the cruciferous Arabidopsis species, Gramineae rice. In addition, the plant may include tobacco, fruits, vegetables, Brassica campestris L. and the like. More specifically, the plants include (but not limited to): wheat, barley, rye, rice, corn, sorghum, sugar beet, apple, pear, plum, peach, apricot, cherries, strawberries, raspberries, blackberries, peas, beans, soybeans, rapeseed, mustard, poppy, oleanolic fruit, sunflower, coconut, castor oil plants, cocoa beans, peanuts, gourds, cucumbers, watermelons, cotton, flax, hemp, jute, oranges, lemons, grapes grapefruits, spinaches, the Qing lettuce, asparagus, cabbage, Chinese cabbage, cabbage, carrots, onions, potatoes, tomatoes, green peppers, avocados, cinnamon, camphor, tobacco, nuts, coffee, eggplant, sugar cane, tea, pepper, grape vine, hops, bananas, rubber trees and ornamental plants.

In preferred embodiments of the present invention, as "plant" is a "crop." The term "crops" means plants of economic value in the food, cotton, oil, etc. agriculture and industry. Their economic value lies in their seeds or biomass. Crops include, but are not limited to: dicotyledons or monocotyledons. Preferred monocotyledons are Gramineae, more preferably rice, wheat, barley, corn, sorghum, etc. Preferred dicotyledonous plants include, but are not limited to: the Malvaceae cotton genus, Cruciferae Brassica genus, more preferably cotton, rapeseed, Arabidopsis thaliana, etc.

As used herein, plant "traits" include, but are not limited to: seed volume, seed weight, seed fiber length, seed fiber strength, plant branch number, plant fruiting number, plant biomass and/or plant yields

As used herein, "improvement of plant traits", " improved traits", "improved plant traits", "trait improvement" or "plant traits improvement" are interchangeable and should means that after improvement with embodiments of the invention, as compared to before improvement, the seed volume is increased, seed weight is increased, the seed fiber is longer, seed fiber is stronger, the plant branch number increases, plant fruiting number increases, the plant biomass increases and/or the plant yield increases.

### Yield

The term "yield" of a plant may relate to vegetative biomass (root and/or shoot biomass), to reproductive organs, and/or to propagates (such as seeds) of that plant.

The term "yield related trait" includes but not limited to seed volume, seed weight, seed fiber strength, the plant branch number, plant fruiting number, plant biomass and/or plant yield.

### Increase/Improve/Enhance

The terms "increase", "improve" or "enhance" are interchangeable and shall mean in the sense of the application at least a 2%, 3%, 4%, 5%, 6%, 7%, 8%, 9% or 10%, preferably at least 15% or 20%, more preferably 25%, 30%, 35% or 40% more yield and/or growth or other agronomic traits in comparison to control plants as defined herein.

As used herein, the term "seed index" or "the weight of one hundred seeds" are used interchangeably to refer to the weight of one hundred seeds, which reflects the seed sizes and fillings.

As used herein, the term "promoter" or "promoter region (domain)" or "regulatory element", "control sequence" are all used interchangeably herein and are to be taken in a broad context to refer to regulatory nucleic acid sequences capable of effecting expression of the sequences to which they are ligated. Promoter is usually present upstream (5 'end) of the target gene coding sequence and can induce the transcription of the nucleic acid sequence into mRNA. In general, the promoter or the promoter region provides the recognition site for RNA polymerase and other factors necessary for the initiation of correct transcription. Different types of promoters, such as constitutive promoter, inducible promoter, development regulating promoter or tissue or organ-specific promoter, can be chosen according to different use by those who skilled in the art. Under the regulation of a tissue or organ-specific promoter, gene transcription occurs only in the specific organ or tissue.

As used herein, the "operably linked" or " operable link" refers to the arrangement of two or more nucleic acid regions or nucleic acid sequences in a functional manner. For example: when the promoter region is placed at a specific location relative to the target gene nucleic acid sequence, transcription of the nucleic acid sequence can be induced by the promoter region. Thus, the promoter region is "operably linked" to the nucleic acid sequence.

As used herein, the term "transgenic plants (crops)," "transformants," or "transgenic plant" are used interchangeably. These terms all refer to plant cells, organs or plants that have been transfected with any of the two genes of the present invention.

### Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be an individual missing the transgene by segregation. A "control plant" as used herein refers not only to whole plants, but also to plant parts, including seeds and seed parts.

### Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

### Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest.

### RDL1 gene and protein

As used herein, term *"RDL1* gene", "RDL1 polypeptide coding gene", "RDL1 polypeptide coding polynucleotide" are all used interchangeably herein and refer to a sequence that is highly homologous to the sequence coding for the cotton RDL1 protein, or a molecule that can hybridize with the above described sequences under stringent conditions, or a family gene molecule that is highly homologous with the above molecule. The expression of these genes can improve plant traits, such as seed volume increases, weight increase, fiber length increases, and/or fiber strength increases. The definition also includes the molecular that can hybridize with cotton RDL1 gene sequence under stringent conditions, or a family gene member that is highly homologous with the above molecules.

As used herein, the term "cotton *RDL1* gene" refers to a sequence that is highly homologous with the sequence encoding the cotton RDL1 protein, the definition include molecules that can hybridize with the cotton RDL1 gene sequence under stringent conditions, or a family gene member that is highly homologous with the above described molecules. Preferably, the gene is specifically and highly expressed during cotton fiber elongation period, such as the Gossypium hirsutum RDL1 coding genes (GhRDL1), which encodes a 335 amino acid residue protein, which contains a plant-specific BURP domain at the C-terminus.

NCBI has published the RDL1 sequence and its homologous gene sequences, such as AY072821 (Li C-H, Gossypium hirsutum dehydration-induced protein RD22-like protein (RDL) mRNA, complete cds); AY641990 (Wang S, of Gossypium arboreum dehydration-induced protein RD22-like protein 1 (RDL1) mRNA, RDL1-1 of allele, complete cds); AY641991 (Wang, S of Gossypium arboreum dehydration-induced protein RD22-like protein 2 (RDL2) mRNA, RDL2-2 of allele, complete cds). These genes are within the scope of the present invention.

RDL1 gene of the present invention may be selected from: (a) SEQ ID NO: 1, SEQ ID NO: 3, or SEQ ID NO: 5 (respectively corresponding to AY072821, AY641990 and AY641991); or (b) a molecule that can hybridize with the sequence defined in (a) under stringent conditions and possesses an activity to improve plant traits. As used herein, the term "stringent conditions" means: (1) hybridization and wash under low ionic strength and high temperature, such as 0.2 × SSC, 0.1% SDS, 60°C; or (2) hybridization in the presence of a denaturant, such as 50% (v/v) formamide, 0.1% calf serum/0.1% of the Ficoll, 42°C, etc.; or (3) hybridization occurs only when the identities between the two sequences are at least 50%, preferably 55 %, more than 60%, 65%, 70%, 75%, 80%, 85% or 90%, more preferably more than 95%. For example, the sequence may be a complementary sequence to the sequence defined in (a).

Full-length nucleic acid sequence or fragments of RDL1 gene of the present invention can often be obtained using PCR amplification, recombinant techniques, or synthetic methods. With respect to PCR amplification, one can use the nucleic acid sequences disclosed in the present invention, particularly the open reading frame sequences, to design primers, and use a commercially available cDNA library or cDNA library prepared with conventional methods known to one skilled in the art as a template, to amplify the related sequences. When the sequence is long, often two or more PCR amplifications are performed, and then the amplified fragments are spliced together in the correct order

It should be understood that the RDL1 gene according to the present invention preferably is obtained from cotton, or other gene sequence from other plants highly homologous with the cotton RDL1 gene (e.g., with sequence identities of 50% or more, preferably 55% or more, 60% or more, 65% or more, 70% or more, 75 % or more, 80% or more, more preferably 85% or more, e.g., 85%, 90%, 95%, or even 98%) are also considered equivalent to the cotton RDL1 gene in present invention. The methods and tools for sequence comparison and identity determination are also well known in the art, such as BLAST

In the present invention, the term "RDL1 protein" refers to a polypeptide encoded by RDL1 gene of the invention. The definition also includes the variants of the above polypeptide having plant trait improving function. Proteins of the present invention can be purified natural product, or a product of chemical synthesis or produced from prokaryotic or eukaryotic hosts (for example, bacteria, yeast, higher plant, insect and mammalian cells) using recombinant techniques. Preferably, the RDL1 proteins of the present invention are encoded by cotton RDL1 gene or its homologous genes or family genes. The RDL1 protein sequences of the present invention may be selected from: (a) SEQ ID NO: 2, SEQ ID NO: 4, or SEQ ID NO: 6; or (b) a protein variant derived from one or more amino acid substitutions, deletions, or additions in the sequences defined in (a) and having plant trait improving functions.

The variations include (but are not limited to): one or more (usually 1-50, preferably 1-30, more preferably, 1-20, most preferably 1-10, for example, 1, 2, 3, 4, 5, 6, 7, 8, 9, or 10) amino acid deletions, insertions and/or substitutions, as well as addition of one or several (usually 20 or less, preferably 10 or less, more preferably 5 or less) amino acids at the C-terminus and/or N-terminus. For example, in the field, substitutions with amino acids with similar properties or similar amino acids, usually do not change the protein functions. Conserved substitution of amino acid is well known in the field of art (refer to Creighton (1984) proteins. W. H. Freeman and Company). For example, adding one or more amino acids at the C-terminus and/or N terminus usually will not change the protein functions. For example, RDL1 proteins of the present invention may or may not include the initial methionine residue and still have the plant trait improving functions.

One can use radiation or exposure to mutagens to induce random mutagenesis. One may also use site-directed mutagenesis or other known molecular biology techniques to obtain the protein described in (b) above. One can use the sequence encoding the protein to construct transgenic plants, and screen and identify the proteins by observing whether there is any improvement in the plant traits (for example, referring to the methods in the working examples in this description).

Depending on the hosts used in the recombinant production of the proteins, proteins of the present invention may be glycosylated or may be non-glycosylated. The term also includes protein fragments and derivatives of RDL1 protein that retain the activities.

The variant forms of the polypeptides include: homologous sequences, conservative variants, allelic variants, natural mutants, induced mutants, proteins encoded by sequences that can hybridize with RDL1 sequence under high or low stringency conditions, and proteins or polypeptides obtained using an anti-RDL1 antiserum. The present invention can also use other polypeptides, such as fusion proteins containing RDL1 protein or its fragments. In addition to the almost full-length polypeptide, the present invention also includes soluble RDL1 protein fragments. Typically, a fragment contains a sequence, from the RDL1 protein sequence, of at least about 10 consecutive amino acids, usually at least about 30 consecutive amino acids, more preferably at least about 50 consecutive amino acids, more preferably at least about 80 consecutive amino acids, and most preferably at least about 100 consecutive amino acids.

### EXPA1 gene and protein

As used herein, *"EXPA1* gene", "EXPA1 polypeptide coding gene", "EXPA1 polypeptide coding polynucleotide" are all used interchangeably herein and refer to a gene highly homologous to the sequence encoding the cotton EXPA1 protein, or a molecular that can hybridize with the above-described gene sequence under stringent conditions, or a molecule in the family of genes that are highly homologous with the above-described molecule. The expression of such gene can confer certain improvements in the plant traits, such as the increase in size, weight increase, increased fiber length and/or increased fiber strength. The definition also includes a molecular that can hybridize with cotton EXPA1 gene sequences under stringent conditions, or a molecule in the family of genes that are highly homologous with the above-described molecules.

As used herein, the term "the cotton *EXPA1* gene" refers to a sequence that is highly homologous to the sequence encoding cotton EXPA1 protein. The definition include a molecular that can hybridize with the cotton EXPA1 gene sequence under stringent conditions, or a molecule in the family genes that are highly homologous with the above-described molecules

NCBI has published the *EXPA1* and its homologous gene sequences, such as AF043284, DQ204495 (Gossypium hirsutum alpha-expansin 1). These genes are within the scope of the present invention.

An EXPA1 gene according to the present invention may be selected from: (a) SEQ ID NO: 7 or SEQ ID NO: 9 (which are respectively corresponding to AF043284 or DQ204495); or (b) a molecule that can hybridize with the sequence defined in (a) under stringent conditions and possess a plant trait improvement function. As used herein, the term "stringent conditions" means: (1) hybridization and wash under low ionic strength and high temperature, such as 0.2 × SSC, 0.1% SDS, 60°C; or (2) hybridization in the presence of a denaturant, such as 50% (v/v) formamide, 0.1% calf serum/0.1% Ficoll, 42°C, etc.; or (3) hybridization occurs only when the identity between the two sequences is at least 50% or more, preferably 55% or more, 60% or more, 65% or more, 70% or more, 75% or more, 80% or more, 85% or more, or 90% or more, more preferably 95% or more. For example, the sequence may be a complementary sequence to that described in (a).

The full-length nucleic acid sequence or fragments of EXPA1 gene according to the invention can be obtained by PCR amplification, recombinant techniques, or synthetic methods. For PCR amplification, one can design primers according to the nucleic acid sequence disclosed in the present invention, in particular the open reading frame sequence, and use a commercially available cDNA library or a cDNA library prepared by conventional methods known to one skilled in the art as a template, to amplify the related sequence. When the sequence is long, twice or more PCR amplifications are often performed, and then the amplified fragments are spliced together in correct order.

It should be understood that the EXPA1 gene according to the present invention preferably is obtained from cotton, or other gene sequence from other plants highly homologous with the cotton EXPA1 gene (e.g., with sequence identities of 50% or more, preferably 55% or more, 60% or more, 65% or more, 70% or more, 75 % or more, 80% or more, more preferably 85% or more, e.g., 85%, 90%, 95%, or even 98%) are also considered equivalent to the cotton EXPA1 gene in present invention. The methods and tools for sequence comparison and identity determination are also well known in the art, such as BLAST. More preferably, comparison is taken under default parameters.

In the present invention, the term "EXPA1 protein" refers to a polypeptide encoded by the EXPA1 gene of the invention. The definition includes variants of the above polypeptide and possessing a function for improving plant traits. Proteins of the present invention may be purified natural product or a product of chemical synthesis or a protein produced from a prokaryotic or eukaryotic host (for example, bacteria, yeast, higher plant, insect and mammalian cells) using recombinant techniques. Preferably, the EXPA1 proteins are encoded by cotton EXPA1 gene or its homologous genes or family genes. The EXPA1 protein sequence of present invention may be selected from: (a) SEQ ID NO: 8 or SEQ ID NO: 10; or (b) a protein derived from the sequence defined in (a) having one or more amino acid substitutions, deletions or insertions and having an activity capable of improving plant traits

The variants include (but are not limited to): one or more (usually 1-50, preferably 1-30, more preferably 1-20, most preferably 1-10, for example, 1, 2, 3, 4, 5, 6, 7, 8, 9, or 10) amino acid deletions, insertions and/or substitutions, as well as one or several (usually 20 or fewer, preferably 10 or fewer, more preferably 5 or fewer) amino acid insertions at the C-terminus and/or N-terminus. For example, as known in the field, substitutions with amino acids having similar properties or with similar amino acids usually do not change the protein functions. For example, additions of one or more amino acids at the C-terminus and/or N terminus usually will not change the protein functions. For example, an EXPA1 protein according to the present invention may or may not include the initiation methionine residue and still has the ability to improve plant traits.

One can use radiation or exposure to mutagens to produce random mutagenesis. One can also use site-directed mutagenesis or other known molecular biology techniques to obtain the protein described in (b) above. One can use a sequence encoding the protein to construct transgenic plants, and to screen and identify the proteins by observing whether they can improve the plant traits (for example, referring to methods disclosed in the working example in the invention).

According to the hosts used in the recombination techniques, a protein of the present invention may be glycosylation or may be non-glycosylation. The term also includes EXPA1 protein fragments and derivatives that retain the activities.

The variants of the polypeptides include: homologous sequences, conservative variants, allelic variants, natural mutants, induced mutants, proteins encoded by sequences that can hybridize with EXPA1 sequence under high or low stringency condition, and polypeptides or proteins obtained using anti-EXPA1 antiserum. The present invention can also use other polypeptides, such as fusion proteins containing EXPA1 protein or its fragments. In addition to almost full-length polypeptides, polypeptides according to the present invention also include EXPA1 active fragments. Typically, such fragment include at least about 10 consecutive amino acids, usually at least about 30 consecutive amino acids, preferably at least about 50 consecutive amino acids, more preferably at least about 80 consecutive amino acids, and most preferably at least about 100 consecutive amino acids in EXPA1 protein sequence. Preferably, the fragments include the consecutive amino acid residues at positions 197-258 in EXPA1 protein sequence, which is the RDL1 interaction site, suggesting that this fragment possesses EXPA1 biological activity and has similar biological functions as that of the full-length EXPA1 protein. More preferred fragments include the continuous amino acid residues at positions 197-258 in the protein sequence of SEQ ID NO 8 or SEQ ID NO.10.

### Constructs and host

In order to specifically improve plant traits, especially seed traits and biomass, the present inventors have conducted extensive studies to find suitable genes for plant trait improvement and made the corresponding constructs.

Therefore, the present invention provides a construct, which includes: an RDL1 protein expression cassette and an EXPA1 protein expression cassette. The expression cassette includes all necessary components for gene expression (including a promoter, coding DNA, as well as a terminator, etc.), thereby the corresponding protein can be expressed. The RDL1 protein expression cassette and the EXPA1 protein expression cassette may be in the same construct or in different constructs. Preferably, the RDL1 protein expression cassette and the EXPA1 protein expression cassette are in the same construct such that it would be simpler to transfect cells.

Typically, the constructs are constructed in an expression vector. Therefore, the present invention also includes vectors, which contain the constructs described. The expression vectors typically contain a replication origin and/or a marker gene. Methods well known to one skilled in the art can be used to construct the desired expression vectors of the present invention. These methods include in vitro recombinant DNA technology, DNA synthesis, in vivo recombination technology, etc. The DNA sequences can be effectively coupled to an appropriate promoter in the expression vector to direct mRNA synthesis. The expression vector also includes the ribosome binding site for translation initiation and a transcription terminator. The present invention preferably uses pEGFP-1, pCAMBIA1300, pCAMBIA2301, or pBI121, and so on. In specific examples of the present invention, the recombinant vector is the pCAMBIA series vectors

In addition, the expression vectors preferably contain one or more selection marker genes to provide phenotypic characteristics for selecting transformed host cells, e.g., for eukaryotic cells: dihydrofolate reductase, neomycin resistance and green fluorescent protein (GFP), or for E. coli: tetracycline or ampicillin resistance.

When expressing polynucleotides of the invention in higher eukaryotes, if enhancer sequences are inserted in the vector, the transcription will be enhanced. Enhancers are cis-acting elements in DNA, usually about 10-300 base pairs. The roles of promoters are to enhance gene transcriptions. One of ordinary skill in the art would know how to select appropriate carriers, promoters, enhancers, and host cells.

Vectors containing the appropriate nucleotide sequences and promoters or control sequences may be used to transfect appropriate hosts. In accordance with methods of the present invention, the hosts may be any suitable hosts for the expression vectors and for transferring the expression vectors to plant cells. Preferably, the hosts are Agrobacteria. As a means, a method for preparing transgenic plants is as follows: transfecting an expression vector carrying a construct into an Agrobacterium, and then using the Agrobacterium, integrating a fragment containing the construct into a chromosome of a plant.

Plant transfections may use Agrobacterium-mediated transformation or gene gun transformation methods, such as leaf disk methods. The transformed plant cells, tissues or organs can be re-grown into plants using conventional methods, resulting in plants with improved traits. The transformants may be cultured using conventional methods to express the polypeptides encoded by genes of the present invention. Depending on the host cells used, media used in cultures may be selected from a variety of conventional media to grow the cultures under conditions suitable for host cell growth. When the host cells have grown to an appropriate cell density, one can use appropriate methods (such as temperature switch or chemical induction) to induce the selected promoter and culture the cells for a further period of time.

### Methods for improving plant traits

The present invention further provides a method for producing crop seeds with improved traits. The method may comprise: enhancing the expression level of *RDL1* gene and *EXPA1* gene in the crops. That is, the improvement is achieved by enhancing RDL1 gene expression levels in the crops, or enhancing the RDL1 protein and EXPA1 of protein levels. One skilled in the art can choose an appropriate improvement method according to the purpose, such as gene transfection, which usually includes the steps of constructing a vector carrying the *RDL1* gene and *EXPA1* gene, transforming a plant, and breeding the transformed plant.

In accordance with a particularly preferred embodiment of this invention, a method may use Agrobacterium-mediated transformation technology to introduce constructs into a plant (such as the callus of a plant).

To perform these methods, one can use any suitable conventional means, including reagents, temperatures, and pressure conditions.

The present invention also includes a plant using the aforementioned method. The plant may include: a transgenic plant with the construct transfected therein; or a plant having up-regulated expression levels of *RDL1* gene and *EXPA1* gene in the cells (including high expression or overexpression), and so on.

One can select, from these transgenic plants, seeds having up-regulated expression levels of *RDL1* gene and *EXPA1* gene to generate offsprings of the plants (e.g., hybrid offsprings) that also have up-regulated expression levels of *RDL1* gene and *EXPA1* gene.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The following description uses specific examples to further illustrate the present invention. It should be understood that these examples are for illustration only and should not be used to limit the scope of the invention. In the following examples, methods that are without specific experimental conditions are usually performed under conventional conditions, such as those described in Sambrook et al, Molecular Cloning: Laboratory Manual (New York: Cold Spring Harbor Laboratory Press), or in accordance with manufacturers recommended conditions. Unless otherwise noted, the percentages and fraction numbers are based on weight.

Unless otherwise defined, all technical and scientific terms used herein have the same meanings as understood by one skilled in the art. In addition, any methods and materials that are similar or equal to those described herein may also be used with embodiments of the invention. The preferred methods and materials described herein are for demonstration only.

In order to further study the function of the RDL1 and improve the quality and yield of cotton, to provide the molecular basis of cotton breeding, the present inventors used yeast two-hybrid methods to screen for GhRDL1-interacting proteins and obtained a cotton α-expansin protein, GhEXPA1. Similar to *GhRDL1, GhEXPA1* is also highly expressed during the period of rapid cotton fiber elongation. The interactions between GhRDL1 and GhEXPA1 are confirmed by further yeast two-hybrid experiments and bimolecular fluorescence complementation assay (BiFC). The present inventors have overexpressed *GhRDL1* and *GhEXPA1,* separately or simultaneously, in Arabidopsis and cotton to produce high-yield phenotypes. Particularly, when these two genes are coexpressed, the transgenic plants grow well and produce more flowers and more fruits. The yield improvement is apparent. For example, in the transgenic cottons, the cotton boll numbers are increased, cotton fiber lengths are increased, and the seed cotton yields increase. In transgenic Arabidopsis thaliana, the silique numbers per plant increase, and the seed numbers, seed sizes and seed weights are significantly increased.

### Example 1, GhRDL1 and GhEXP1 cDNA isolation

Cotton RNA was extracted by cold phenol. Cotton materials (2 g) were ground into powder in liquid nitrogen and transferred to a 50 ml centrifuge tube. An extraction buffer (8 ml, 1 M Tris•HCl, 50 mM EDTA, 1% SDS, pH 9.0) and an equal volume of water-saturated phenol:chloroform:isoamyl alcohol (25:24:1) were added. The mixture was mixed well and placed on ice for 1 h, with stirring every 10 min. This was then centrifuged for 20 min at 4°C, 13000 g. Repeat the extraction with phenol:chloroform:isoamyl alcohol 2 to 4 times, and finally extract with chloroform:isoamyl alcohol (24:1) once. Supernatant was collected, to which 1/2 volume of a high salt solution (0.8 M sodium citrate, 1.2 M NaCl) and 1/2 volume of isopropanol were added. The mixture was mixed and stored at -70°C for 1 h. It was then centrifuged at 4°C and 13000 g for 20 min. The supernatant was discarded, and the precipitate was dissolved in 1 ml of DEPC processed water. It was centrifuged at 4°C and 13000 g for 10 min. The supernatant was transferred to 1.5 ml Eppendorf tube, and 1/3 volume of 8 M of LiCl and 1/10 volume of NaAC were added. The tube was stored at -20°C overnight, and then centrifuged at 4°C and 13000 g for 20 min. The supernatant was discarded. The precipitate was washed with 1 ml of 70% ethanol 2 times, air dried for 20 min at room temperature, and then dissolved in 100 ~ 200 µL of DEPC processed water.

Based on the sequences of *GhRDL1* and *GhEXP1* cDNA (GenBank accession No: AY072821 and DQ204495, respectively), specific primers (including restriction sites and protecting bases) were synthesized:
RDL1-F-PstI: 5'-AACTGCAGGGAATTAGTCACTCCTGTTCTA-3'(SEQ ID NO: 11),
RDL1-R-*Kpn*I: 5'-GGGGTACCGATTTCACATAACTAAACTCGG-3' (SEQ ID NO: 12);
EXP1-F-*Nco*I: 5'-CATGCCATGGGTCAGCCAATTGTTTGAGCTAGC-3' (SEQ ID NO: 13),
EXP1-R-*BamH*I-*BstE*II:
5'-GGGTTACCGGATCCCTACCTCGGCATAAAACGCTCA-3' (SEQ ID NO: 14),

9-DPA ("9-DPA" means 9 days post anthesis of cotton) fiber total RNA reverse transcription product were used as the template for PCR amplification. The reaction conditions were: 94°C pre-denaturation for 5 min; then 94°C denaturation at 30 s, 56°C renaturation for 30 s, and 72°C extension for 1 min for 35 cycles, and a final extension at 72°C for 10 min. After subcloning, the products were sequenced to confirm the correct sequences.

### Example 2, using yeast two-hybrid method to screen for GhRDL1-interacting proteins

Proteins that interact with a target protein are screened by yeast two-hybrid method using the MATCHMAKER library construction kit (Clontech).

### pGBKT7-GhRDL1 construct:

*GhRDL1* gene (ATG-TAA complete coding sequence) was amplified using KOD-Plus high-fidelity polymerase. At the same time, *EcoR*I*-BamH*I restriction sites were introduced. After checking the reading frame, *GhEXPA1* fragments were excised with *EcoR*I and *BamH*I digestions and annealed to the corresponding sites in the *pGBKT7* vector (purchased from Clontech) to form *pGBKT7-GhRDL1.*

In *pGADT7-Rec* vector (purchased from Clontech), both sides flanking the SmaI restriction site contain recombination sequences of SMART III and CDS III. Total RNA from cotton fibers at 6 days after flowering (anthesis) was subjected to reverse transcription (reverse transcription system adds the SMART III chain 5'-AAGCAGTGGTATCAACGCAGAGTGGCCATTA TGGCCGGG-3' (SEQ ID NO: 21) and CDS III reverse transcriptase primer 5'-ATTCTAGAGGCCGAGGCGGCCGACATG-d(T)₃₀VN-3' (SEQ ID NO: 22) N = A, G, C, or T; V = A, G or C) to generate double-stranded cDNA (ds cDNA) library, which was amplified to produce a modified cDNA library using primers (5' PCR primer: 5'-TTCCACCCAAGCAGTGGTATCAACGCAGAGTGG-3' (SEQ ID NO: 23) and 3' PCR primer: 5'-GTATCGATGCCCACCCTCTAGAGGCCGAGGCGGCCGA CA-3'(SEQ ID NO: 24)). The modified cDNA library and *pGBKT7-GhRDL1,* and linearized (single cut with SmaI) *pGADT7-Rec* vector (containing the same recombination site) were used together to transform yeast AH109 strain (from Clontech). In the yeast, the *PGADT7-Rec* vector and the cDNA library recombined to form vector *pGADT7-Rec-*cDNA. Yeasts co-transformed with *pGBKT7-GhRDL1* and *pGADT7-Rec-*cDNA were grown with the SD/-Thr/-Leu/-His/-Arg medium (refers to Thr, Leu, His, Arg deficient medium) to screen for positive clones.

In an experiment using yeast two-hybrid method to screen for GhRDL1 interacting proteins, 59 positive clones were obtained, of which 14 were sequenced. Six clones containing a gene fragment encoding α-expansin protein (starting at the 591bp, counting from the ATG start codon, to the poly A sequence, which corresponds to the 197-258 amino acid sequence of□ α-expansin protein (EXPA1)). The gene is *GhEXPA1.*

### Example 3, Construction of 35S::GhRDL135S::GhEXPA1 co-expression vectors (RE) and Agrobacterium transformation

The *GhRDL1* and *GhEXPA1* target fragments were amplified with KOD-Plus high-fidelity polymerase with simultaneous introduction of corresponding restriction enzyme cleavage site (see Example 1 for the primer designs). After checking for the reading frame, the *GhEXPA1* fragment was excised with *Nco*I and *BstE*II double digestion and constructed into the corresponding sites in the *pCAMBIA1301* vector (purchased from CAMBIA) to produce of the intermediate vector *(E), p1301-EXP1,* which was verified by sequencing. The *GhRDL1* fragment excised with *Pst*I and *Kpn*I double digestion was constructed into a modified *pCAMBIA2301* vector (*pCAMBIA2301* purchased from CAMBIA, the modifications are as follows: introduction, at the *Hind*III*-Pst*I and *Sac*I*-EcoR*I sites in the multi-cloning sites, of 35S (which was obtained by PCR amplification of the pBI121 vector with introduction of the *Hind*III*-Pst*I site. The pBI121 vector was purchased from Clontech) and NOS (which was obtained by PCR amplification of the pBI121 vector with introduction of the *Sac*I*-EcoR*I site) to form the intermediate vector (R), *p2301-RDL1,* which was verified by sequencing. The *p1301-EXP1* intermediate vector was cut with *Hind*III and *BstE*II double digestion. The fragment from the digestion (including promoter) was constructed into the *p2301-RDL1* intermediate vector at the corresponding site to form the final expression vector *35S::GhRDL1 35S::GhEXPA1* (*RE,* Figure 2).

The transformation of Agrobacterium was performed with the freeze-thaw method. A single colony of LBA4404 or GV3101 (Invitrogen) in 3 ml of LB medium (25 µg/ml rifamycin (Rif) and 50 µg/ml of kanamycin (Kan) or gentamicin (Gen)) was cultured at 28°C and 220 rpm overnight. 2 ml of culture in 50 ml of LB medium (25 µg/ml of Rif and 50 µg/ml of Gen) was cultured at 28°C and 220 rpm to OD600 = 0.5 (about 6 h). Place the culture on ice for 30 min, and then centrifuge it at 4°C and 5000 g for 5 min. Resuspended the pellet in 10 ml of 0.15 M NaCl and centrifuged at 4°C and 5000 g for 5 min. Resuspended the pellet in 1 ml of 20 mM and CaCl₂; aliquot it at 50 µl/tube; quickly freeze them in liquid nitrogen; and preserve the competent cells at -70°C. Mix the aforementioned constructs of expression vectors containing any target gene and 50 µl/tube of competent cells; place the tube on ice for 30 minutes; quickly freeze it in liquid nitrogen for 1 min; thaw the culture in a 37°C water bath for 5 min; add 1 ml LB medium; culture at 28°C and 220 rpm for 2 to 4 h; take a 50 ~ 100 µl aliquot and streak an LB plate (25 µg/ml Rif, 50 µg/ml Gen, and 50 µg/ml Kan or hygromycin (Hyg)). After 2 days, pick single colonies and analyze by PCR.

### Example 4, Plant transformation and screening of transgenic offspring

### a. Cotton genetic transformation

The Agrobacterium carrying the plasmid were cultured in YEB bacteria culture medium containing kanamycin (50 mg/L), rifampicin (100 mg/L), streptomycin (300 mg/L) for 3 days. Then, a single colony was picked and inoculated in YEB liquid medium containing the same antibiotics. It was grown in the suspension culture in a shaker at 28°C and 200 rpm/min overnight. The culture was then centrifuged at 4000 rpm/min for 10 min. The precipitate was resuspended in 1/2 MS liquid medium containing glucose 30 g/L and acetosyringone 100 µmol/L. The culture was adjusted to OD₆₀₀ of about 0.4 to 0.6 and left for later tranfections.

After routine disinfection, cotton R15 seeds (a tetraploid wild-type upland cotton, as transgenic parent) was added onto 1/2MS0 culture medium (1/2MS salt +5 g/L glucose +7 g/L agar, pH 6.0) and cultured in the dark to allow germination. After 5 to 7 days, the sterile hypocotyl was cut into sections about 1.0 cm in size for later use as transformation explants.

Explants were immersed in the solution containing Agrobacterium bacteria for infection for 15-20 min, and then transferred to a co-culture medium MSB1 (MS salts + B5 organic + 30 g/L glucose + 0.1 mg/L KT + 0.1 mg/L 2,4-D + 2.2 g/L Gelrite, pH 6.0) and cultured at 22°C in the dark for 2 days. The explants were transferred to the MSB2 medium (MSB1 +500 mg/L cefotaxime + 80 mg/L kanamycin) to induce callus formation. After callus induction, callus proliferation, and embryogenic callus induction (medium MSB3: MS salt + B5 organic + 30 g/L glucose + 2.5 g/L Gelrite, pH 6.0), somatic embryogenesis (medium MSB4: MS salt + B5 organic + 30 g/L glucose + 1.0 g/L asparagine + 2.0 g/L glutamine + 3.0 g/L Gelrite, pH 6.0; the MS salt contains double amount of KNO₃. but without NH₄NO₃), the explant regenerated resistant plantlets. When the plantlets had grown to 3-4 true leaves, transplant them to pots and allow them to grow in a climate controlled room.

### b. Genetic transformation of Arabidopsis

Arabidopsis plants were transformed using the floral dip method (Clough, and of Bent, 1998, Plant J. 16,735-743). Agrobacterium culture methods are as described above. The bacteria culture was centrifuged at 4000 rpm/min for 10 min, and the bacteria were re-suspended in 500 ml 5% sucrose solution containing 0.02% Silwet L-77. The above ground parts of wild-type plants (Col-0) were soaked in the broth for 5 s and then laid flat in a plastic tub. Keep them moist and in the dark for 16 ~ 24 h. The T0 generation seeds were vernalized at 4°C for 2 to 4 days. Then, they were treated in 20% bleach water for 15 min and washed with sterile water 3 to 4 times. They were then suspended in 0.5% agarose (55°C) and laid on top of a 0.6% agar LB medium (50 µg/ml Kan or Hyg) at 22°C, with continuous light. After about one week, the green seedlings resistant to the antibiotics were transplanted into fertile soils (peat moss:vermiculite:perlite, 1:1:1) and allowed to grow.

### Example 5, Molecular biology characterization of transgenic plants

### a. PCR

DNA was extracted by the cold phenol method. 2 g of material was ground to a powder in liquid nitrogen and transferred to a 50 ml centrifuge tube. Add 8 ml extraction buffer (1 M Tris-HCl, 50 mM EDTA, 1% SDS, pH 9.0) and an equal volume of water saturated phenol: chloroform: isoamyl alcohol (25:24:1). The mixture was mixed by shaking and placed on ice for 1 h, while stirring it every 10 min. Then, it was centrifuged at 4°C, 13000 g for 20 min. Repeat the phenol: chloroform: isoamyl alcohol extractions 2 to 4 times, and finally extract with chloroform: isoamyl alcohol (24:1) once. Collect the supernatant and add 1/2 volume of a high salt solution (0.8 M sodium citrate, 1.2 M NaCl) and 1/2 volume of isopropanol. Mix well and keep it at -70°C for 1 h. Then centrifuge at 4°C, 13000 g for 20 min. Discard the supernatant and washed the precipitation with 1 ml of 70% ethanol. It was then allowed to dry at room temperature for 20 min with circulating air. The precipitate was dissolved in 1 ml of sterilized water and centrifuged at 4°C, 13000 g for 10 min. Collect the supernatant, and add 5-10 µl of the RNase (10 mg/ml) and keep it at 37°C for 30 min.

*NPTII* or gene specific primers (for cotton *kanamycin (NPTII)* gene specific primers, the sequences of the primers are: NPTII-F: GGCGATACCGTAAAGCACGAGGAA (SEQ ID NO: 15) and NPTII-R: GCTATGACTGGGCACAACAGACAAT (SEQ ID NO: 16); for Arabidopsis gene specific primers, the sequences are: GhRDL1-RT-F: CAAATACTCCAATGCCAAAG (SEQ ID NO: 17) and GhRDL1-RT-R: GAGTTTCACTGGCTGCATAT (SEQ ID NO: 18); GhEXP1-RT-F : AAGGGTATG GAACGAGCACAG (SEQ ID NO: 19) and GhEXP1-RT-R: CCATCGCTGGCAGTCACTTTA (SEQ ID NO: 20) were used for PCR analysis. The reaction conditions were as follows: 94°C initial denaturation for 5 min; 94°C pre-denaturation 30 s, 56°C renaturation for 30 s, extension at 72°C for 1 s, 35 cycles; final extension at 72° C for 10 s.

The results of electrophoresis of PCR products for several transgenic cotton plants (RE302, RE303, RE305, RE308) and the R15 wild-type plant are shown in Figure 3.

### b. Detection of kanamycin (Kanamycin, Kan) resistance

About three weeks after germination (emergence of 3 true leaves) of transgenic cotton seeds, the new leaves were wiped with 0.5% *Kanamycin* solution. After normal growth for about one week, observed the color of the leaves that had been wiped with *Kanamycin* solution. If the leaves are still green, the plants have Kan resistance, hence the plants are positive for the transgene. If the leaf colors are apparently yellow, the plants do not have Kan resistance, and are negative for the transgene.

### Example 6, Analysis of the traits of transgenic plants

### a. Analysis of transgenic cotton traits

*R* (i.e., *GhRDL1* transgenic cotton; *35S::GhRDL1), E* (i.e., *GhEXPA1* transgenic cotton; 35S::GhEXPA1) and RE (i.e., GhRDL1, and GhEXPA1 transgenic cotton; *35S::GhRDL1 35S::GhEXPA1*) transgenic cottons and wild-type R15 were cultivated in Shanghai Wuku farm (April 2010). Among the T1 generation plants of transgenic lines, those with average growths were photographed. Mature cotton bolls for individual plants were collected. Efforts were made to keep the collection parts as consistent as possible. Randomly pick 100 seeds from mature bolls of each individual plant. Select a predetermined number of seeds, comb their fibers flat, and measure the lengths of the fibers. Then, measure the weights of 100 seeds (without fibers), count the number of branches, and count the boll numbers for each individual plant. Analyze these data with statistics. The data from the statistical analysis were shown in graphs. The T1 generation data, shown in FIG. 4, for Line RE-302 #, RE-303 #, RE-305 # and RE-308 # are averages of five plants. As compared to the wild-type cotton, the transgenic cotton plants *35S::GhRDL1* (*R* series), *35S::GhEXPA1* (*E* series), and the *35S::GhRDL1 35S::GhEXPA1* transgenic cottons (*RE* series) have significant increases in the branch numbers and average cotton boll numbers. In addition, the seed kernel weights and lengths of the cotton fiber are shown in Table 1. As compared to the control, the E series of transgenic cottons and the RE series of transgenic cottons have longer fibers. As compared to the control, the E-series of transgenic plants have increased number of branches.

At the same time, the present inventors also compared the fruiting status of the transgenic cotton plants, as shown in Figure 5. It is apparent that, as compared to the wild-type cotton, the *35S::GhRDL1 35S::GhEXPA1* had a significant increase in the fruiting numbers.

**Table 1, analysis of transgenic cotton fiber lengths and seed index**

| Cotton lines | Weight of 100 seeds¹ | Fiber legth² | p Vlue³ |
|---|---|---|---|
| R15 | 13.13±0.12 | 26.4±0.8 | - |
| R-10# | 13.69±0.25 | 28.4±0.8 | 4E-05 |
| R-117# | 13.10±0.45 | 29.4±0.8 | 1E-07 |
| R-119# | 13.97±0.23 | 30.2±0.6 | 3E-09 |
| E-202# | 11.01±0.58 | 29.3±1.6 | 1E-04 |
| E-213# | 11.96±0.45 | 28.0±0.8 | 3E-04 |
| E-216# | 10.85±0.96 | 27.8±0.6 | 3E-04 |
| E-218# | 14.39±0.16 | 28.4±0.9 | 4E-05 |
| RE-302# | 11.82±0.31 | 26.2±1.2 | 0.758 |
| RE-303# | 13.32±0.30 | 28.9±0.6 | 8E-07 |
| RE-305# | 12.98±0.24 | 29.9±0.9 | 1E-08 |
| RE-308# | 13.88±0.58 | 27.3±0.7 | 0.156 |

| | | | |
|---|---|---|---|
| ¹ The value is for the weight of 100 seeds randomly selected from each line (n = 3). ² The value is for the fiber lengths of different cotton bolls from each line (n = 10). ³ The value is referred to the t-test of the wild-type R-15. | | | |

Even though the weights of 100 seeds did not significantly increase, the total weights of plant seeds increased substantially because the numbers of fruits and the numbers of seeds increased.

Twenty RE transgenic plants of the T1 generation with improved traits were planted at the Experimental Farm of Chinese Cotton Company located in Sanya, Hainan, China. There were about 900 plants in the 0.5 mu farm, including R15, R105, R117, and R119 plants (about 100 plants each). Using kanamycin resistance to distinguish whether the plant had transgene, it was found in the initial screening that the positive response is greater than 50%.

After two and a half months, the plant heights and leave numbers were counted. The result showed that the RE transgenic plants have significant increases in the plant heights and leave numbers (Fig. 6). In addition, some of the individual plants started to germinate, whereas the control R15 plants had no plant in the budding state, indicating that the *RE* transgenic plant has a better growth trend.

After three and a half months, the flower numbers and branch numbers were counted. The results showed that three species of the *RE* transgenic plants have significantly increased flower numbers and branch numbers (Fig. 7). In addition, the inventors also recorded the timing of bud formation (the number of days until the first bud appeared), the timing of the first flower (the number of days until the first flower appeared), and the time of flowering (the number of days when half of the plants have flowers). The results are shown in Table 2. It can be seen that as compared to the R15 plant, the RE transgenic cottons had the buds 1-6 days earlier; the first flower appeared 1-5 earlier, and the flowering time is 2-5 days earlier.

The cotton boll numbers were counted for individual plants after maturation. The results show that the *RE* transgenic plants have substantially increased cotton boll numbers (Fig. 12A). A repeat experiment conducted at Songjiang Farm in Shanghai confirmed these results (Fig. 12B). The cotton plants grown at Songjiang Farm are divided into small areas each as a unit to count the cotton fiber yields. The results showed that some plants also have significant advantages in the cotton fiber yields (Fig. 12C). The RE3-8-7 plant line has a 40% increase in the cotton fiber yield as compared to the control. To understand the cotton fiber qualities, we collected the cotton fibers and sent them to China Cotton Fiber Quality Analysis Center (China Cotton Company, Anyang, Henan, China) for analysis. The results show that the quality of the RE transgenic cotton fibers are generally superior to those of the control (Table 3).

**Table 2, Budding time, initial flowering time, and flowering time for individual transgenic cottons.**

| Cotton lines | Budding time | Initial flowering | Flowering time |
|---|---|---|---|
| R15-1 | 80 | 104 | 109 |
| R15-2 | 80 | 104 | 107 |
| R117 | 77 | 99 | 103 |
| R119 | 77 | 98 | 103 |
| RE3-2-5 | 74 | 99 | 106 |
| RE3-2-6 | 79 | 99 | 103 |
| RE3-3-1 | 78 | 102 | 106 |
| RE3-3-2 | 76 | 102 | 105 |
| RE3-8-8 | 78 | 103 | 108 |
| RE3-8-9 | 74 | 102 | 105 |

**Table 3, Fiber qualities of transgenic cottons**

| | Cotton lines | Fiber length (mm) | Lint percentage (%) | Micronaire | Fiber Strength (cN/tex) |
|---|---|---|---|---|---|
| Sanya | R15 | 26.5 | 35.0 | 5.3 | 26.9 |
| | RE3-2-1 | 26.7* | 38.4** | 4.9 | 27.3 |
| | RE3-2-6 | 26.9* | 39.4** | 4.9 | 27.4 |
| | RE3-3-2 | 27.2 | 33.7** | 4.7 | 29.1 |
| | RE3-5-2 | 27.5 | 33.0** | 4.7 | 28.3 |
| | RE3-8-7 | 27.6* | 38.4** | 4.8 | 29.3* |
| | RE3-8-9 | 27.2** | 39.0* | 4.7 | 28.0 |
| Songjiang | R15 | 27.8 | 29.2 | 5.4 | 27.5 |
| | RE3-2-1 | 28.5 | 34.0** | 5.1 | 28.7** |
| | RE3-2-6 | 27.8 | 32.5** | 5.2 | 28.1* |
| | RE3-3-2 | 28.8* | 27.5 | 4.9 | 28.2** |
| | RE3-5-2 | 29.1* | 28.4 | 5.2 | 27.6 |
| | RE3-8-7 | 28.6 | 32.7* | 5.1 | 28.1* |
| | RE3-8-9 | 27.8 | 33.5** | 5.1 | 27.4 |

| | | | | | |
|---|---|---|---|---|---|
| The values are generated from 20 cotton bolls randomly selected from each line. Statistics are referred to the t-test of the wild-type R15. *: *p*<0.05; **: *p*<0.01 | | | | | |

### b. Analysis of transgenic Arabidopsis thaliana traits

*R*, *E*, and *RE* transgenic vectors were introduced into Arabidopsis thaliana by Agrobacterrium-mediated transformation. Antibiotic resistant T1 plant were selected out from T0 generation transgenic seeds by antibiotic screening and verified the transgene expression level by RT-PCR. T1 generation seeds were harvest from on individual plant. 12 individual plant were picked, and the seeds of these plants (T2 generation plant) were screened again by antibiotic. The plant of which two generations were both resistant to the antibiotic (both grew on the medium with Kan, i.e. the offsprings did not separate) were homozygous positive. Based on resistance screening and RT-PCR methods (Fig. 8), pure T2 or T3 positive transgenic plants were identified.

After harvesting mature Arabidopsis thaliana seeds and drying for 3 days, the seeds were photographed under microscope with 20x amplification (n>50), see Fig. 9. By the ImageJ program (Rasband W., NIH, USA), the seed lengths and widths were measured and statistically analyzed (Fig. 9 and Table 3). The results show that RE transgenic Arabidopsis thaliana has substantially increased seed sizes as compared to the control. Furthermore, the mature seed epithelials of the RE plants also have larger volumes, though there is no significant difference in the appearance. The seed weights were analyzed by batchwise weighing 1000 seeds and repeated 5 times. The results show that the RE transgenic Arabidopsis thaliana had significantly increased seed weights per 1000 seeds; the 1000 seed weight so the transgenic Arabidopsis thaliana were almost twice those of the wild-type control (Table 3). In addition, for E transgenic Arabidopsis thaliana, seed sizes were also substantially larger than the control seeds, the 1000 seed weights were also significantly increased, the fruiting numbers and the seed numbers were also increased.

Healthy siliques from 50-day old Arabidopsis thaliana plant stems were randomly picked to measure the lengths of the siliques and the number of seeds in each silique. At least 5 plants were analyzed for each transgenic plant. A paper bag was placed over a single plant Arabidopsis thaliana to ensure that all seeds were collected. The seeds were dried and weighed to analyze the seed biomass (yields). At least 10 plants were analyzed for each transgenic plant line. It was apparent that the biomass of the *RE* transgenic Arabidopsis thaliana were significantly higher that the wild-type and the R or E transgenic plants.

The above ground parts of 40 or 46 day old Arabidopsis thaliana were collected and weighed (fresh weights and dry weights) for analysis of the biomass under healthy growth. At least 10 plants were analyzed for each transgenic plant line (Fig. 10). It was apparent that the biomass of the *RE* transgenic Arabidopsis thaliana were substantially increased.

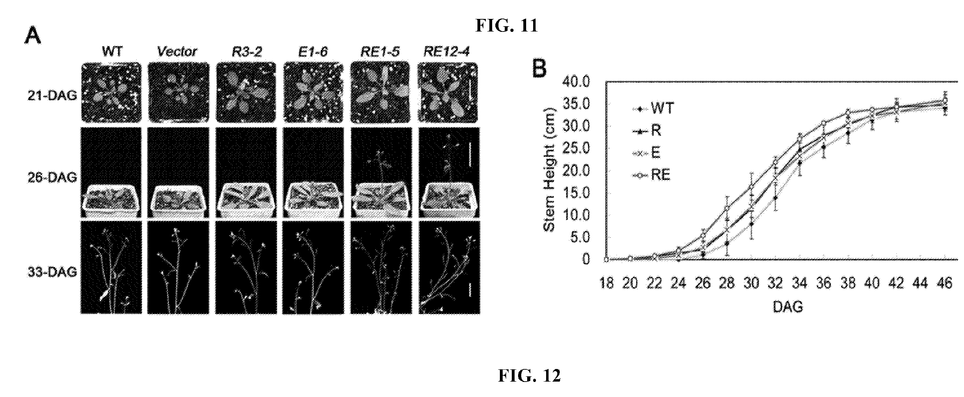

The height changes of 18-46 days (days after germination) old shoots of Arabidopsis thaliana were measured to reflect the growth rates of Arabidopsis thaliana. At least 10 plants were analyzed for each transgenic plant line (Fig. 11). It was apparent that the *RE* transgenic Arabidopsis thaliana growth rates were significantly higher that those of the control.

**Table 3. Analysis of silique numbers and seeds yields of transgenic Arabidopsis thaliana¹ lines.**

| Genotype | Seed size (length x width) mm | *P* value^{2,3} | Weight of 1000 seeds, mg⁴ | *P* value² | Siliques per plant⁵ | *P* value² | Seed yield per plant, mg⁶ | *P* value² |
|---|---|---|---|---|---|---|---|---|
| WT | 0.428±0.040× 0.254±0.022 | -, - | 13.82±0.50 | - | 255.2±46.2 | - | 152±39 | - |
| Vector | 0.434±0.035× 0.258±0.014 | 0.070, 0.139 | 14.08±0.75 | 0.54 | 246.6±18.9 | 0.715 | 145±24 | 0.703 |
| R3-2 | 0.486±0.034× 0.277±0.018 | 8.E-17, 2.E-10 | 17.50±1.46 | 2.E-04 | 316.2±18.2 | 0.039 | 224±26 | 0.002 |
| E1-6 | 0.501±0.054× 0.292±0.024 | 5.E-12, 3.E-14 | 18.32±0.54 | 8.E-07 | 300.2±19.4 | 0.097 | 220±33 | 0.040 |
| RE1-5 | 0.503±0.031× 0.288±0.019 | 1.E-26, 1.E-18 | 18.34±0.36 | 6.E-07 | 396.0±36.4 | 8.E-04 | 299±27 | 4.E-05 |
| RE12-4 | 0.490±0.045× 0.278±0.022 | 7.E-14, 3.E-10 | 16.84±0.42 | 8.E-06 | 415.8±44.5 | 5.E-04 | 271±37 | 4.E-04 |

| | | | | | | | | |
|---|---|---|---|---|---|---|---|---|
| ¹The values are given as mean ± standard deviation; ² T-test values are referred to the wild-type; ³ The P values are for seed length and width, respectively.(n > 100); ⁴The values are generated by randomly weighting 1000 seeds from each line (n = 5); ⁵ The values are generated by counting the siliques numbers at 60-DAG (days after germination) from single plant (n = 10); ⁶The values are generated by weighting total dry seeds from single plant (n = 10). | | | | | | | | |

All references cited herein are incorporated by reference, as if they were individually cited herein. In addition, it should be understood that after reading the disclosure of this invention, one skilled in the art can modify or vary the invention. These modifications and variations are equivalents and are within the scope of the invention as defined in the claims.

## Claims

1. A method for improving a plant trait, the method comprising: increasing expression of *EXPA1* gene or an activity of *EXPA1* polypeptide in the plant.

2. The method according to claim 1, **characterized in that** the method further comprises: increasing expression of *RDL1* gene or an activity of RDL1 polypeptide in the plant.

3. The method according to claim 1, wherein the increasing is achieved by introducing and expressing *EXPA1* gene in the plant.

4. The method according to claim 2, wherein the increasing is achieved by introducing and expressing *RDL1* gene in the plant.

5. The method according to any one of claims 1-4, **characterized in that** the EXPA1 polypeptide is one or more selected from the following:
(a) a polypeptide comprising the amino acid sequence of SEQ ID NO: 8 or 10;
(b) a protein derived from (a) having substitution, deletion, or addition of one or several amino acids in the amino acid sequence defined in (a) and having a function of improving the plant trait; or
the RDL1 polypeptide is one or more selected from the following:
(a1) a polypeptide comprising the amino acid sequence of SEQ ID NO: 2, 4, or 6;
(b1) a polypeptide derived from (a1) having substitution, deletion, or addition of one or several amino acids in the amino acid sequence defined in (a1) and having a function of improving plant traits.

6. The method according to any one of claims 1-4, **characterized in that** the *EXPA1* gene comprises the following polynucleotide sequence:
(a2) the nucleotide sequence of SEQ ID NO: 7 or 9;
(b2) a nucleotide sequence capable of hybridizing with the sequence defined in (a2) under a stringent condition and having a function of improving the plant trait;
(c2) a nucleotide sequence having, in an increasingly preferred order, a sequence identity of 40%, 50%, 60%, 70%, 75%, 80%, 85%, 90%, 92%, 95%, 96%, 98%, or higher to the sequence defined in (a2); or
the *RDL1* gene comprises the following nucleotide sequence:
(a3) a nucleotide sequence of SEQ ID NO: 1, 3, or 5;
(b3) a nucleotide sequence capable of hybridizing with the sequence defined in (a3) under a stringent condition and having a function of improving the plant trait;
(c3) a nucleotide sequence having, in an increasingly preferred order, a sequence identity of 40%, 50%, 60%, 70%, 75%, 80%, 85%, 90%, 92%, 95%, 96%, 98%, or higher to the sequence defined in (a3).

7. The method according to claim 2, **characterized in that** the method comprises:
(1) providing a construct, wherein the construct comprises an EXPA1 polypeptide expression cassette;
(2) introducing the construct into the plant to improve the plant trait.

8. The method according to claim 4, **characterized in that** the method comprises:
(1) providing a construct, wherein the construct comprises an EXPA1 polypeptide expression cassette and an RDL1 polypeptide expression cassette;
(2) introducing the construct into the plant to improve the plant trait,
provided that: the EXPA1 polypeptide expression cassette and the RDL1 polypeptide expression cassette are located on the same construct or located on different constructs.

9. The method according to claim 7 or 8, **characterized in that** the EXPA1 polypeptide expression cassette comprises a promoter sequence, an *EXPA1* gene sequence, and a stop codon; or
the RDL1 polypeptide expression cassette comprises: a promoter sequence, an *RDL1* gene sequence, and a stop codon.

10. The method according to claim 7 or 8, **characterized in that** step (2) comprises:
(a) transforming an Agrobacterium with the construct and obtaining a construct-carrying Agrobacterium; and
(b) contacting a plant cell or a tissue or an organ with the construct-carrying Agrobacterium in step (a), thereby introducing the construct into the plant.

11. The method according to any one of claims 1, 2, and 7, **characterized in that** the improving the plant trait comprises improving a plant yield-related trait.

12. The method according to claim 11, **characterized in that** the improving the plant yield-related trait comprises: increasing seed fiber length, increasing branching number, increasing seed volume, increasing seed weight, increasing fruiting number, and/or increasing yield.

13. The method according to claim 11, **characterized in that** the improving the plant yield-related trait comprises: increasing seed volume, increasing seed weight, increasing seed fiber length, increasing seed fiber strength, increasing branching number, increasing flowering, increasing fruiting number, enhancing growth rate, accelerating germination, increasing biomass, and/or increasing yield.

14. A transgenic plant, or a hybrid offspring thereof, obtained with the method according to any one of claims 1-13, wherein when compared with a control plant, the transgenic plant, or the hybrid offspring thereof, has an improved yield-related trait

15. The transgenic plant, or the hybrid offspring thereof, according to claim 14, **characterized in that** the improved trait comprises: increased seed volume, increased seed weight, increased seed fiber length, increased seed fiber strength, increased branching number, increased flowering, increased fruiting number, enhanced growth rate, accelerated germination, increased biomass, and/or increased yield.

16. A construct, wherein the construct comprises: an EXPA1 polypeptide expression cassette.

17. The construct according to claim 16, **characterized in that** the construct further comprises: an RDL1 polypeptide expression cassette.

18. Use of the *EXPA1* gene or a combination of the *EXPA1* gene and the *RDLI* gene or the construct according to claim 16 or 17 in the production of a plant with an improved trait.

19. A host cell, **characterized in that** the host cell contains the construct according to claim 16 or 17.

20. Use of a transgenic plant produced by the method according to any one of claims 1-13 for the production of a plant seed having an improved trait.

21. The use according to claim 20, **characterized in that** the improved trait comprises: increased seed volume, increased seed weight, increased seed fiber length, and/or increased seed fiber strength.

22. A gene composition used for improving a plant trait, comprising *RDLI* gene and *EXPA1* gene.


Sequence Listing

<110>  Shanghai Institute for Biological Sciences, Chinese Academy of
Sciences

<120>  Genetic Engineering Methods and Materials for Improving Plant Yields

<130>  107072

<160>  24

<170>  PatentIn version 3.3

<210>  1
<211>  1297
<212>  DNA
<213>  Upland cotton(Gossypium hirsutum)

<400>  1
ggcacgaggg aattagtcac tcctgttcta gatgaaggtt ctctccccaa ttcttgcttg      60
cctagcgctt gctgtggtgg taagccatgc tgctctctca cctgagcaat attggagcta     120
taagctgcca aatactccaa tgccaaaggc tgtcaaagaa attctacatc cagaactgat     180
ggaggagaaa agcacctctg taaatgtagg aggtggtggt gtaaacgtca atacaggaaa     240
agggaagcct gggggtgaca cccatgtgaa cgttggaggc aaaggagttg gagtgaacac     300
gggaaagcca gggggtggca ctcatgtgaa tgttggagac ccttttaatt acctatatgc     360
agccagtgaa actcaaatcc atgaagaccc gaatgtggct cttttctttc tggaaaagga     420
tatgcacccc ggggcaacaa tgagcctgca tttcactgaa aatacagaga aatcagcttt     480
cttaccttat caaactgccc aaaaaatacc gttttcatct gacaagttgc cagaaatttt     540
caacaagttt tcagtgaaac ctggatcact gaaggcagag atgatgaaga acacaattaa     600
ggagtgcgaa cagccagcga ttgaaggaga ggaaaaatat tgtgcaacct cactggagtc     660
aatgattgac tatagcattt ccaaactagg gaaagttgat caggcagtct caacagaagt     720
ggaaaaacaa accccaatgc aaaagtatac aatagcagct ggagtgcaga agatgacaga     780
tgacaaagct gtagtgtgcc acaagcagaa ttatgcatat gctgtcttct attgccataa     840
atcagaaaca acaagggctt acatggttcc tttagagggt gctgacggaa caaaagccaa     900
agcagtagca gtctgccaca cagatacatc agcatggaac cctaagcatt tggcttttca     960
agtcctaaaa gttgaaccag gaaccattcc tgtctgccat ttccttcctc gggatcacat    1020
tgtttgggtc cctaagtaaa aatcctgaag agtagattca tacactataa gtttcatcat    1080
agtgtgcatt aaaacagctt aaagcaaaat ccagtttgtt ttataataaa aaacccccga    1140
gtttagttat gtgaaatcta tccatgaatc atgtttttgg taatggataa aatgatatta    1200
ctttttgtat cccaaaggtt tggtgataaa tgtattagta tttgaagtat cgttgcagaa    1260
aaaaaaaaaa aaaaaaaaaa aaaaaaaaaa aaaaaaa                             1297

<210>  2
<211>  335
<212>  PRT
<213>  Upland Cotton(Gossypium hirsutum)

<400>  2

Met Lys Val Leu Ser Pro Ile Leu Ala Cys Leu Ala Leu Ala Val Val
1               5                   10                  15
Val Ser His Ala Ala Leu Ser Pro Glu Gln Tyr Trp Ser Tyr Lys Leu
            20                  25                  30
Pro Asn Thr Pro Met Pro Lys Ala Val Lys Glu Ile Leu His Pro Glu
        35                  40                  45
Leu Met Glu Glu Lys Ser Thr Ser Val Asn Val Gly Gly Gly Gly Val
    50                  55                  60
Asn Val Asn Thr Gly Lys Gly Lys Pro Gly Gly Asp Thr His Val Asn
65                  70                  75                  80
Val Gly Gly Lys Gly Val Gly Val Asn Thr Gly Lys Pro Gly Gly Gly
                85                  90                  95
Thr His Val Asn Val Gly Asp Pro Phe Asn Tyr Leu Tyr Ala Ala Ser
            100                 105                 110

Glu Thr Gln Ile His Glu Asp Pro Asn Val Ala Leu Phe Phe Leu Glu
        115                 120                 125
Lys Asp Met His Pro Gly Ala Thr Met Ser Leu His Phe Thr Glu Asn
    130                 135                 140
Thr Glu Lys Ser Ala Phe Leu Pro Tyr Gln Thr Ala Gln Lys Ile Pro
145                 150                 155                 160
Phe Ser Ser Asp Lys Leu Pro Glu Ile Phe Asn Lys Phe Ser Val Lys
                165                 170                 175
Pro Gly Ser Leu Lys Ala Glu Met Met Lys Asn Thr Ile Lys Glu Cys
            180                 185                 190
Glu Gln Pro Ala Ile Glu Gly Glu Glu Lys Tyr Cys Ala Thr Ser Leu
        195                 200                 205
Glu Ser Met Ile Asp Tyr Ser Ile Ser Lys Leu Gly Lys Val Asp Gln
    210                 215                 220
Ala Val Ser Thr Glu Val Glu Lys Gln Thr Pro Met Gln Lys Tyr Thr
225                 230                 235                 240
Ile Ala Ala Gly Val Gln Lys Met Thr Asp Asp Lys Ala Val Val Cys
                245                 250                 255
His Lys Gln Asn Tyr Ala Tyr Ala Val Phe Tyr Cys His Lys Ser Glu
            260                 265                 270
Thr Thr Arg Ala Tyr Met Val Pro Leu Glu Gly Ala Asp Gly Thr Lys
        275                 280                 285
Ala Lys Ala Val Ala Val Cys His Thr Asp Thr Ser Ala Trp Asn Pro
    290                 295                 300
Lys His Leu Ala Phe Gln Val Leu Lys Val Glu Pro Gly Thr Ile Pro
305                 310                 315                 320
Val Cys His Phe Leu Pro Arg Asp His Ile Val Trp Val Pro Lys
                325                 330                 335

<210>  3
<211>  1307
<212>  DNA
<213>  Asian Cotton(Gossypium arboreum)

<400>  3
ccattccatg caacactaac ttttagattc cctttccatt gcactgctct ttctttctat      60
agaattagtc actcctgttc tagatgaagg ttctctcccc aattcttgct tgcctagcgc     120
ttgctgtggt ggtaagccat gctgctctct cacctgagca atattggagc tataagctgc     180
caaatactcc aatgccaaag gctgtcaaag aaattctaca tccagaactg atggaggaga     240
aaagtacctc tgtaaatgta ggaggtggtg gtgtaaacgt caatacagga aaagggaagc     300
ctgggggtga cacccatgtg aacgttggag gcaaaggagt tggagtgaac acgggaaagc     360
cagggggtgg cactcatgtg aatgatccag acccttttaa ttacctatat gcagccagtg     420
aaactcaaat ccatgaggac ccgaatgtgg ctcttttctt tctggaaaag gatatgcacc     480
ccggggcgac aatgagcctg catttcattg aaaatacaga gaaatcagct ttcttacctt     540
atcaaactgc ccaaaaaaat acgttttcat ctgacaagtt gccagaaatt ttcaacaagt     600
tttcagtgaa acctggttca gtgaaggcag agatgatgaa gaacacaatt aaggagtgcg     660
aacagccagc gattgaagga gaggaaaaat attgtgcaac ctcactggag tcaatgattg     720
actatagcat ttccaaacta gggaaagttg atcaggcagt ctcaacagaa gtggaaaaac     780
aaaccccaat gcaaaagtat acaatagcag ctggagtgca gaagatgaca gatgacaaag     840
ctgtagtgtg ccacaagcag aattatgcat atgctgtctt ctattgccat aaatcagaaa     900
caacaagggc ttacatggtt cctttagagg gtgctggcgg aacaaaagcc aaagcactag     960
cagtctgcca cacagataca tcagcatgga acccaaagca tttggctttt caattcctaa    1020
aagttgaacc aggaaccatt cctgtctgtc atttccttcc tcgggatcac attgtttggg    1080
tccctaagta aaaatcctga agagtagact catacactat agtttcatca tagtgtgcat    1140
taaaacagct taaagcaata tccagtttgt tctataataa tatatccagg agtttagtta    1200
tgtaaaatct atccatgaat catgttcttg gtaatggata aaatgatatt acttcccgta    1260
tcacaatggt ttggtgataa atgtattagt attttaagta tctttgc                  1307

<210>  4
<211>  335
<212>  PRT
<213>  Asian Cotton(Gossypium arboreum)

<400>  4

Met Lys Val Leu Ser Pro Ile Leu Ala Cys Leu Ala Leu Ala Val Val
1               5                   10                  15
Val Ser His Ala Ala Leu Ser Pro Glu Gln Tyr Trp Ser Tyr Lys Leu
            20                  25                  30
Pro Asn Thr Pro Met Pro Lys Ala Val Lys Glu Ile Leu His Pro Glu
        35                  40                  45
Leu Met Glu Glu Lys Ser Thr Ser Val Asn Val Gly Gly Gly Gly Val
    50                  55                  60
Asn Val Asn Thr Gly Lys Gly Lys Pro Gly Gly Asp Thr His Val Asn
65                  70                  75                  80
Val Gly Gly Lys Gly Val Gly Val Asn Thr Gly Lys Pro Gly Gly Gly
                85                  90                  95
Thr His Val Asn Asp Pro Asp Pro Phe Asn Tyr Leu Tyr Ala Ala Ser
            100                 105                 110
Glu Thr Gln Ile His Glu Asp Pro Asn Val Ala Leu Phe Phe Leu Glu
        115                 120                 125
Lys Asp Met His Pro Gly Ala Thr Met Ser Leu His Phe Ile Glu Asn
    130                 135                 140
Thr Glu Lys Ser Ala Phe Leu Pro Tyr Gln Thr Ala Pro Lys Asn Thr
145                 150                 155                 160
Phe Ser Ser Asp Lys Leu Pro Glu Ile Phe Asn Lys Phe Ser Val Lys
                165                 170                 175
Pro Gly Ser Val Lys Ala Glu Met Met Lys Asn Thr Ile Lys Glu Cys
            180                 185                 190
Glu Gln Pro Ala Ile Glu Gly Glu Glu Lys Tyr Cys Ala Thr Ser Leu
        195                 200                 205
Glu Ser Met Ile Asp Tyr Ser Ile Ser Lys Leu Gly Lys Val Asp Gln
    210                 215                 220
Ala Val Ser Thr Glu Val Glu Lys Gln Thr Pro Met Gln Lys Tyr Thr
225                 230                 235                 240
Ile Ala Ala Gly Val Gln Lys Met Thr Asp Asp Lys Ala Val Val Cys
                245                 250                 255
His Lys Gln Asn Tyr Ala Tyr Ala Val Phe Tyr Cys His Lys Ser Glu
            260                 265                 270
Thr Thr Arg Ala Tyr Met Val Pro Leu Glu Gly Ala Gly Gly Thr Lys
        275                 280                 285
Ala Lys Ala Leu Ala Val Cys His Thr Asp Thr Ser Ala Trp Asn Pro
    290                 295                 300
Lys His Leu Ala Phe Gln Phe Leu Lys Val Glu Pro Gly Thr Ile Pro
305                 310                 315                 320
Val Cys His Phe Leu Pro Arg Asp His Ile Val Trp Val Pro Lys
                325                 330                 335

<210>  5
<211>  1430
<212>  DNA
<213>  Asian Cotton(Gossypium arboreum)

<400>  5
ccattccatg caacactaac ttttagattc cctttccatt gtactgatct ttctttcttt      60
agaattagtc actcctgttc tagatgaagg ttctctcccc aattcttgct tgcctagcgc     120
ttgctgtggt ggcaagtcat gctgctctct cacccgagca atattggagc tataagctgc     180
caaatactcc aatgccaaag gctgtcaaag aaattctaca tccagaactg atggaggaga     240
aaagtacctc tgtaaatgta ggaggtggtg gtgtaaacgt caacacagga aaagggaagc     300
cagcgggtgg cactcatgtg aacgttgggc gcaaaggagt tggagtgaac acgggaaagc     360
cagggggtgg cactcatgtg aatgttggag gcaaaggagt tggggtgaac actggaaagc     420
caggaggtgg cacccatgtg aacgttggag gcaaaggtgg aggagtatct gtacacaccg     480
gacacaaggg aaagccagta aatgttaatg tgagtccgtt tctttaccaa tatgcagcca     540
gtgaaactca aatccatgac gatccgaatg tggctctttt ctttctggaa aaggatttac     600
accccggggc aacaatgagc ctgcatttca ctgaaaatac agagaaatcc gctttcttac     660
cttatcaaac tgcccaaaaa ataccgtttt catctaacga gttgccagaa attttcaaca     720

agttttcagt gaaacctgga tcagtgaagg cagagatgat gaagaacaca attaaggagt 780
gcgaacagcc agcgattgaa ggagaggaaa aatattgtgc aacctcactg gagtcaatga 840
ttgactacag catttccaaa ctagggaaag ttgatcaggc agtctcaaca gaagtggaaa 900
aacaaacccc aacgcacaag tatacaataa cagctggagt gcagaagatg acaaatgaca 960
aagctgtagt gtgccacaag cagaattatg catatgctgt cttctattgc cataaatcag 1020
aaacaacaag ggcttacatg gttcctttag agggtgctga cggaacaaaa gccaaagcag 1080
tagcagtctg tcacacagat acatcagcat ggaacccaaa gcatttggct tttcaagtcc 1140
taaaagttga gccaggaacc attcctgtct gccatttcct tcctcgggat cacattgttt 1200
gggtccccaa gtaaaagtcc tgaagagtag actcatacac tatagtttca tcatagggtg 1260
cattaaaaca gcttaaagca atctccagtt tgttctataa taatataccc acgagtttag 1320
tcatgtaaaa tctatccatg aatcatgttc ttagtaatgg ataaaatgat agtactttct 1380
gtatcacaag ggtctggcga taaatttatt agtattttaa gtaacttttc 1430

<210> 6
<211> 376
<212> PRT
<213> Asian Cotton(Gossypium arboreum)

<400> 6

Met Lys Val Leu Ser Pro Ile Leu Ala Cys Leu Ala Leu Ala Val Val
1               5                   10                  15
Ala Ser His Ala Ala Leu Ser Pro Glu Gln Tyr Trp Ser Tyr Lys Leu
            20                  25                  30
Pro Asn Thr Pro Met Pro Lys Ala Val Lys Glu Ile Leu His Pro Glu
        35                  40                  45
Leu Met Glu Glu Lys Ser Thr Ser Val Asn Val Gly Gly Gly Gly Val
    50                  55                  60
Asn Val Asn Thr Gly Lys Gly Lys Pro Ala Gly Gly Thr His Val Asn
65                  70                  75                  80
Val Gly Arg Lys Gly Val Gly Val Asn Thr Gly Lys Pro Gly Gly Gly
                85                  90                  95
Thr His Val Asn Val Gly Gly Lys Gly Val Gly Val Asn Thr Gly Lys
            100                 105                 110
Pro Gly Gly Gly Thr His Val Asn Val Gly Gly Lys Gly Gly Gly Val
        115                 120                 125
Ser Val His Thr Gly His Lys Gly Lys Pro Val Asn Val Asn Val Ser
    130                 135                 140
Pro Phe Leu Tyr Gln Tyr Ala Ala Ser Glu Thr Gln Ile His Asp Asp
145                 150                 155                 160
Pro Asn Val Ala Leu Phe Phe Leu Glu Lys Asp Leu His Pro Gly Ala
                165                 170                 175
Thr Met Ser Leu His Phe Thr Glu Asn Thr Glu Lys Ser Ala Phe Leu
            180                 185                 190
Pro Tyr Gln Thr Ala Gln Lys Ile Pro Phe Ser Ser Asn Glu Leu Pro
        195                 200                 205
Glu Ile Phe Asn Lys Phe Ser Val Lys Pro Gly Ser Val Lys Ala Glu
    210                 215                 220
Met Met Lys Asn Thr Ile Lys Glu Cys Glu Gln Pro Ala Ile Glu Gly
225                 230                 235                 240
Glu Glu Lys Tyr Cys Ala Thr Ser Leu Glu Ser Met Ile Asp Tyr Ser
                245                 250                 255
Ile Ser Lys Leu Gly Lys Val Asp Gln Ala Val Ser Thr Glu Val Glu
            260                 265                 270
Lys Gln Thr Pro Thr His Lys Tyr Thr Ile Thr Ala Gly Val Gln Lys
        275                 280                 285
Met Thr Asn Asp Lys Ala Val Val Cys His Lys Gln Asn Tyr Ala Tyr
    290                 295                 300
Ala Val Phe Tyr Cys His Lys Ser Glu Thr Thr Arg Ala Tyr Met Val
305                 310                 315                 320
Pro Leu Glu Gly Ala Asp Gly Thr Lys Ala Lys Ala Val Ala Val Cys
                325                 330                 335
His Thr Asp Thr Ser Ala Trp Asn Pro Lys His Leu Ala Phe Gln Val

340                 345                 350
Leu Lys Val Glu Pro Gly Thr Ile Pro Val Cys His Phe Leu Pro Arg
        355                 360                 365
Asp His Ile Val Trp Val Pro Lys
    370                 375

<210>  7
<211>  1102
<212>  DNA
<213>  Upland Cotton(Gossypium hirsutum)

<400>  7
ttagctagct cttactcaaa tggcaaccaa aacgatgatg ttgcaaatat ttccactttt      60
cttctttttg ttcagtgtct gcaactccat tttccttggt gctaatggag atgacaatgg     120
tggttggcaa actgcccatg ccaccttcta cggtggtgct gatgctaccg gcacaatggg     180
gggagcttgt ggttatggaa acctgtacag tcaagggtat ggaacgagca cagcagcttt     240
gagcactgca cttttcaaca atggcttgag ctgcggtgcc tgctacgagc tccggtgcaa     300
caatgatcct caatggtgca ttagtcgaac cataaccgtg acagccacca acttttgtcc     360
acctaactat gctttatcta gtgacaatgg cgggtggtgc aatcccccac gagaacactt     420
tgatttggcc gaaccgcgat tcttgcggat agcagaatat cgagctggaa tcgtccctgt     480
tatgttcaga agggtgtcat gtgtgaagaa aggaggcatc aggtacacca tgaatggaca     540
ttcgtacttc aacatggtgt tgataacgaa gctgggaggg gcaggggata taacgtcagt     600
gtccatcaag ggttccagaa caggatggct acctatgtcc agaaattggg gccaaaactg     660
gcagagcaat gcttacctta acggacaaag cctctctttt aaagtgactg ccagcgatgg     720
caggactatc acagcctaca atgtagtgcc tgctggttgg caattcggac aaacttttga     780
aggaggccag ttttaagaca atattatagt gtctgtctaa tataaaactg gaattgacat     840
attacttata taaggcacat gagcgtttta tgccgaggta gcaaaatggc cgccgctggc     900
tttatgtgtg aaataggcga gcaagtgcca ttagcctata atctatacat ttcttatagt     960
gaaccaaact attaagtttg aactctagag gatatatcca taatgtctga atttgtttgt    1020
tgatgattga ccatgatatt tatggttttc attattgaat accttttttt ataatttaaa    1080
aaaaaaaaaa aaaaaaaaaa aa                                             1102

<210>  8
<211>  258
<212>  PRT
<213>  Upland Cotton(Gossypium hirsutum)

<400>  8

Met Ala Thr Lys Thr Met Met Leu Gln Ile Phe Pro Leu Phe Phe Phe
1               5                   10                  15
Leu Phe Ser Val Cys Asn Ser Ile Phe Leu Gly Ala Asn Gly Asp Asp
            20                  25                  30
Asn Gly Gly Trp Gln Thr Ala His Ala Thr Phe Tyr Gly Gly Ala Asp
        35                  40                  45
Ala Thr Gly Thr Met Gly Gly Ala Cys Gly Tyr Gly Asn Leu Tyr Ser
    50                  55                  60
Gln Gly Tyr Gly Thr Ser Thr Ala Ala Leu Ser Thr Ala Leu Phe Asn
65                  70                  75                  80
Asn Gly Leu Ser Cys Gly Ala Cys Tyr Glu Leu Arg Cys Asn Asn Asp
                85                  90                  95
Pro Gln Trp Cys Ile Ser Arg Thr Ile Thr Val Thr Ala Thr Asn Phe
            100                 105                 110
Cys Pro Pro Asn Tyr Ala Leu Ser Ser Asp Asn Gly Gly Trp Cys Asn
        115                 120                 125
Pro Pro Arg Glu His Phe Asp Leu Ala Glu Pro Arg Phe Leu Arg Ile
    130                 135                 140
Ala Glu Tyr Arg Ala Gly Ile Val Pro Val Met Phe Arg Arg Val Ser
145                 150                 155                 160
Cys Val Lys Lys Gly Gly Ile Arg Tyr Thr Met Asn Gly His Ser Tyr
                165                 170                 175
Phe Asn Met Val Leu Ile Thr Lys Leu Gly Gly Ala Gly Asp Ile Thr
            180                 185                 190

Ser Val Ser Ile Lys Gly Ser Arg Thr Gly Trp Leu Pro Met Ser Arg
        195                 200                 205
Asn Trp Gly Gln Asn Trp Gln Ser Asn Ala Tyr Leu Asn Gly Gln Ser
    210                 215                 220
Leu Ser Phe Lys Val Thr Ala Ser Asp Gly Arg Thr Ile Thr Ala Tyr
225                 230                 235                 240
Asn Val Val Pro Ala Gly Trp Gln Phe Gly Gln Thr Phe Glu Gly Gly
                245                 250                 255
Gln Phe

<210>  9
<211>  1113
<212>  DNA
<213>  Upland Cotton(Gossypium hirsutum)

<400>  9
gccaattgtt tgagctagct agctcttact caaatggcaa ccaaaacgat gatgttgcaa      60
atatttccac ttttcttctt tttgttcagt gtctgcaact ccattttcct tggtgctaat     120
ggagatgaca atggtggttg gcaaactgcc catgccacct tctacggtgg tgctgatgct     180
accggcacaa tggggggagc ttgtggttat ggaaacctgt acagtcaagg gtatggaacg     240
agcacagcag ctttgagcac tgcacttttc aacaatggct tgagctgcgg tgcctgctac     300
gagctccggt gcaacaatga tcctcaatgg tgcattagtc gaaccataac cgtgacagcc     360
accaactttt gtccacctaa ctatgcttta tctagtgaca atggcgggtg gtgcaatccc     420
ccacgagaac actttgattt ggccgaaccg gcattcttgc ggatagcaga atatcgagct     480
ggaatcgtcc ctgttatgtt cagaagggtg tcatgtgtga agaaaggagg catcaggtac     540
accatgaatg gacattcgta cttcaacatg gtgttgataa cgaacgtggg aggggcaggg     600
gatataacgt cagtgtccat caagggttcc agaacaggat ggctacctat gtccagaaat     660
tggggccaaa actggcagag caatgcttac cttaacggac aaagcctctc ttttaaagtg     720
actgccagcg atggcaggac tatcacagcc tacaatgtag tgcctgctgg ttggcaattc     780
ggacaaactt ttgaaggagg ccagttttaa gacaatatta tagtgtctgt ctaatataaa     840
actggaattg acatattact tatataaggc acatgagcgt tttatgccga ggtagcaaaa     900
tggcgcccgc tggctttatg tgtgaaatag gcgagcaagt gccattagcc tataatctat     960
acttttctta tagtgaacca aactattaag tttgaactct agaggatata tccataatgt    1020
ctgaaatttg tttgttgatg attgaccatg atatttatgc ttttcattat tgaaatacct    1080
ttttttataa tttataaaaa aaaaaaaaaa aaa                                  1113

<210>  10
<211>  258
<212>  PRT
<213>  Upland cotton(Gossypium hirsutum)

<400>  10

Met Ala Thr Lys Thr Met Met Leu Gln Ile Phe Pro Leu Phe Phe Phe
1               5                   10                  15
Leu Phe Ser Val Cys Asn Ser Ile Phe Leu Gly Ala Asn Gly Asp Asp
            20                  25                  30
Asn Gly Gly Trp Gln Thr Ala His Ala Thr Phe Tyr Gly Gly Ala Asp
        35                  40                  45
Ala Thr Gly Thr Met Gly Gly Ala Cys Gly Tyr Gly Asn Leu Tyr Ser
    50                  55                  60
Gln Gly Tyr Gly Thr Ser Thr Ala Ala Leu Ser Thr Ala Leu Phe Asn
65                  70                  75                  80
Asn Gly Leu Ser Cys Gly Ala Cys Tyr Glu Leu Arg Cys Asn Asn Asp
                85                  90                  95
Pro Gln Trp Cys Ile Ser Arg Thr Ile Thr Val Thr Ala Thr Asn Phe
            100                 105                 110
Cys Pro Pro Asn Tyr Ala Leu Ser Ser Asp Asn Gly Gly Trp Cys Asn
        115                 120                 125
Pro Pro Arg Glu His Phe Asp Leu Ala Glu Pro Ala Phe Leu Arg Ile
    130                 135                 140
Ala Glu Tyr Arg Ala Gly Ile Val Pro Val Met Phe Arg Arg Val Ser
145                 150                 155                 160

Cys Val Lys Lys Gly Gly Ile Arg Tyr Thr Met Asn Gly His Ser Tyr
                165                 170                 175
Phe Asn Met Val Leu Ile Thr Asn Val Gly Gly Ala Gly Asp Ile Thr
            180                 185                 190
Ser Val Ser Ile Lys Gly Ser Arg Thr Gly Trp Leu Pro Met Ser Arg
        195                 200                 205
Asn Trp Gly Gln Asn Trp Gln Ser Asn Ala Tyr Leu Asn Gly Gln Ser
    210                 215                 220
Leu Ser Phe Lys Val Thr Ala Ser Asp Gly Arg Thr Ile Thr Ala Tyr
225                 230                 235                 240
Asn Val Val Pro Ala Gly Trp Gln Phe Gly Gln Thr Phe Glu Gly Gly
                245                 250                 255
Gln Phe

<210>  11
<211>  30
<212>  DNA
<213>  Artificial Sequence

<220>
<221>  misc_feature
<223>  Primer

<400>  11
aactgcaggg aattagtcac tcctgttcta                                        30

<210>  12
<211>  30
<212>  DNA
<213>  Artificial Sequence

<220>
<221>  misc_feature
<223>  Primer

<400>  12
ggggtaccga tttcacataa ctaaactcgg                                        30

<210>  13
<211>  33
<212>  DNA
<213>  Artificial Sequence

<220>
<221>  misc_feature
<223>  Primer

<400>  13
catgccatgg gtcagccaat tgtttgagct agc                                    33

<210>  14
<211>  36
<212>  DNA
<213>  Artificial Sequence

<220>

<221>  misc_feature
<223>  Primer

<400>  14
gggttaccgg atccctacct cggcataaaa cgctca                                36


<210>  15
<211>  24
<212>  DNA
<213>  Artificial Sequence


<220>
<221>  misc_feature
<223>  Primer

<400>  15
ggcgataccg taaagcacga ggaa                                             24


<210>  16
<211>  25
<212>  DNA
<213>  Artificial Sequence


<220>
<221>  misc_feature
<223>  Primer

<400>  16
gctatgactg ggcacaacag acaat                                            25


<210>  17
<211>  20
<212>  DNA
<213>  Artificial Sequence


<220>
<221>  misc_feature
<223>  Primer

<400>  17
caaatactcc aatgccaaag                                                  20


<210>  18
<211>  20
<212>  DNA
<213>  Artificial Sequence


<220>
<221>  misc_feature
<223>  Primer

<400>  18
gagtttcact ggctgcatat                                                  20

<210>  19
<211>  21
<212>  DNA
<213>  Artificial Sequence

<220>
<221>  misc_feature
<223>  Primer

<400>  19
aagggtatgg aacgagcaca g                                                21

<210>  20
<211>  21
<212>  DNA
<213>  Artificial Sequence

<220>
<221>  misc_feature
<223>  Primer

<400>  20
ccatcgctgg cagtcacttt a                                                21

<210>  21
<211>  39
<212>  DNA
<213>  Artificial Sequence

<220>
<221>  misc_feature
<223>  Primer

<400>  21
aagcagtggt atcaacgcag agtggccatt atggccggg                             39

<210>  22
<211>  59
<212>  DNA
<213>  Artificial Sequence

<220>
<221>  misc_feature
<223>  Primer

<220>
<221>  misc_feature
<222>  (59)..(59)
<223>  n is a, c, g, or t

<400>  22
attctagagg ccgaggcggc cgacatgttt tttttttttt tttttttttt tttttttvn      59

<210>  23
<211>  33

<212>  DNA
<213>  Artificial Sequence

<220>
<221>  misc_feature
<223>  Primer

<400>  23
ttccacccaa gcagtggtat caacgcagag tgg                                    33

<210>  24
<211>  39
<212>  DNA
<213>  Artificial Sequence

<220>
<221>  misc_feature
<223>  Primer
<400>  24
gtatcgatgc ccaccctcta gaggccgagg cggccgaca                              39